# EUROPEAN PATENT APPLICATION

(11) **EP 3 909 601 A1**
(43) Date of publication of application: **17.11.2021**
(21) Application number: 20173899.4
(22) Date of filing: 11.05.2020
(51) Int. Cl.: A61K 39/00, C07K 16/30

(54) **A NOVEL ANTIBODY BINDING SPECIFICALLY TO HUMAN CEACAM1/3/5 AND USE THEREOF**

(71) Applicant: LeukoCom GmbH, 45147 Essen (DE)
(72) Inventor: Singer, Bernhard B., 45149 Essen (DE)
(74) Representative: Gulde & Partner

(57) **Abstract**

The present invention is directed to an isolated antibody or antigen binding fragment thereof that binds within huCEACAM1/3/5 specifically to the amino acid epitope sequence PQQLFGYSWY (SEQ ID NO: 11), wherein the isolated antibody or antigen binding fragment preferably comprises three heavy chain complementarity determining regions (HCDR1, HCDR2, and HCDR3), and three light chain complementarity determining regions (LCDR1, LCDR2, and LCDR3), wherein HCDR1 comprises the amino acid sequence of SEQ ID NO:1; HCDR2 comprises the amino acid sequence of SEQ ID NO:2; and HCDR3 comprises the amino acid sequence of SEQ ID NO:3; and wherein LCDR1 comprises the amino acid sequence of SEQ ID NO:4; LCDR2 comprises the amino acid sequence of GAT or GATX as in SEQ ID NO:5; and LCDR3 comprises the amino acid sequence of SEQ ID NO:6.

## Description

### INTRODUCTION:

The transmembrane protein carcinoembryonic antigen-related cell adhesion molecule 1 (CEACAM1, also known as biliary glycoprotein (BGP), CD66a and C-CAM1), is a member of the carcinoembryonic antigen family (CEA) that also belongs to the immunoglobulin superfamily. CEACAM1 interacts with other known CEACAM proteins, including CEACAM1 (CD66a), CEACAM6 (CD66c), CEACAM8 (CD66b) and CEACAM5 (CD66e, often only abbreviated as CEA) proteins. It is expressed on a wide spectrum of cell types, ranging from epithelial and endothelial cells to those of hematopoietic origin (e.g. immune cells).

It was shown that the CEACAM1 protein can be over expressed in tumor cells of gastric, lung and thyroid cancer as well as malignant melanoma, but often appears down-regulated in colorectal, liver, breast, prostate and bladder cancer. Additional data support the central involvement of CEACAM1 expression during angiogenesis, metastasis and tumor progression. Many different functions have been attributed to the CEACAM1 protein including CEACAMs participating in multiple physiological and pathological processes including cell-to-cell communication, cell-adhesion, epithelial differentiation, migration, apoptosis and regulation of pro-inflammatory reactions. CEACAM1 also plays a central role in the modulation of innate and adaptive immune responses which they regulate through formation of homophilic and heterophilic interactions. For example, on the one hand CEACAM1 was shown to be an inhibitory receptor for activated T cells contained within the human intestinal epithelium (see e.g. WO99/52552 A1). On the other hand soluble CEACAM8, a physiological ligand of the membrane-anchored CEACAM1-receptor protein has been found to act as a potent co-stimulator of B- and T-cells (US 8,501,192,B2). Additional reports have indicated that CEACAM1 engagement either by T Cell Receptor cross-linking with distinct monoclonal antibodies (mAbs) or by *Neisseria gonorrhoeae* Opa proteins inhibits T cell activation and proliferation. Similar inhibitory effects were seen with other CEACAM binding pathogen-adhesins like HopQ of *Helicobacter pylori* and CbpF of *Fusobacter spp..*

CEACAM1 is not expressed by normal melanocytes, but frequently found on melanoma cells. There is evidence that overexpression of CEACAM1 can be correlated with poor prognosis and is detected in the majority of metastatic melanoma cases (see e.g. WO 2013/054331 A1). CEACAM1 expression on primary cutaneous melanoma lesions strongly predicts the development of metastatic disease with poor prognosis. Moreover, increased CEACAM1 expression was observed on NK cells derived from patients with metastatic melanoma compared with healthy donors.

Evidence indicates that CEACAM1 plays an important role during virus infections. For example, it has been demonstrated that lymphocytes isolated from the deciduae of CMV-infected patients express the CEACAM1 protein in increased levels. The increased CEACAM1 expression on the decidual lymphocytes might diminish the local immune response and serve as another mechanism developed by the virus to avoid recognition and clearance primarily by activated decidual lymphocytes. Further, it was shown that CEACAM1 immunostaining is significantly increased in high-grade squamous intraepithelial lesions (SIL) in comparison with low-grade SIL and normal cervical tissues. It was suggested that CEACAM1 upregulation is related to integration of HPV DNA in high-grade SIL and that CEACAM1 is an important biological marker in SIL and cervical cancer progression. Altogether this evidence indicates that CEACAM1 plays an important role in various viral infections. In addition, CEACAM1 over-expression may serve as marker of various viral infections.

US Patent Application No. 20080108140 discloses methods of modulating *specific* immune responses to create a protective immunity in the treatment of autoimmune diseases and diseases requiring the transplantation of tissue. In particular, it relates to the suppression of immune responses in a targeted fashion, by increasing the functional concentration of the CEACAM1 protein in the target tissue. U.S. Patent Application No. 20040047858 discloses specific antibodies which are capable of modulating T cell activity via CEACAM1 and uses thereof in treating immune response related diseases (e.g. graft versus host disease, autoimmune diseases, cancers etc.).

In WO 2018/015468 A1 it is disclosed that CEACAM1 plays an important role as receptor for human *Helicobacter pylori (H. pylori*) isolates and that binding of *H. pylori* is dependent on interaction of the bacterial surface-exposed adhesin HopQ with human CEACAM1, CEACAM3, CEACAM5 and CEACAM6 but not CEACAM8. It was suggested that *H. pylori* infection can be inhibited by interfering with the specific interaction between bacterial HopQ and human CEACAMs like CEACAM1, CEACAM3, CEACAM5 and/or CEACAM6. Important to note, sole gastric epithelium during gastritis, gastric ulcer and gastric cancer express CEACAMs (namely CEACAM1,5,6) while normal gastric epithelium appears to be CEACAM1, 5, 6 negative. Thus, CEACAM over-expression may also serve as marker of bacterial infections.

Thus, CEACAM1 is involved in a number of different interactions and signalling pathways involved in numerous diseases and medical conditions. Therefore, there still remains a need to identify novel antibodies recognizing specific subsets of CEACAM proteins or the ones cross-reactive with different CEACAMs in a common, functional crucial sections/part/epitope which can be used diagnostically and therapeutically in diseases involving CEACAM expression, organisation (dimer/oligomer versus monomer) or activation.

### DESCRIPTION OF THE INVENTION:

The present invention is directed to an isolated antibody or antigen binding fragment thereof that binds to human CEACAM1, human CEACAM3 and human CEACAM5 (huCEACAM1/3/5), wherein the isolated antibody or antigen binding fragment of the invention binds specifically to an epitope on huCEACAM1/3/5 comprising or consisting of the amino acid sequence of SEQ ID NO:11.

Preferably, the isolated antibody or antigen binding fragment of the invention comprises three heavy chain complementarity determining regions (HCDR1, HCDR2, and HCDR3), and three light chain complementarity determining regions (LCDR1, LCDR2, and LCDR3), wherein:
i) HCDR1 comprises or consists of the amino acid sequence of GYTFTTYV (SEQ ID NO:1);
ii) HCDR2 comprises or consists of the amino acid sequence of FNPYNDGT (SEQ ID NO:2); and
iii) HCDR3 comprises or consists of the amino acid sequence of ARWAYDGSYAY (SEQ ID NO:3); and wherein
iv) LCDR1 comprises or consists of the amino acid sequence of DHINNW (SEQ ID NO:4);
v) LCDR2 comprises or consists of the amino acid sequence of GAT or GATX (SEQ ID NO:5, wherein X at position 4 can be any amino acid); and
vi) LCDR3 comprises or consists of the amino acid sequence of QQYWRTPFT (SEQ ID NO:6).

The inventors have identified a novel antibody that specifically binds to human CEACAM1, in particular to the N-domain of the extracellular part of human CEACAM 1, while said antibody does not bind to CEACAM1 of mouse, rat, bovine or canine origin. Further, the inventors have shown that the antibody of the invention or the antigen binding fragment thereof does bind specifically to an amino acid sequence of PQQLFGYSWY (SEQ ID NO: 11), comprising amino acid residues 59 to 68 of human CEACAM 1, wherein human CEACAM 1 has the amino acid sequence of SEQ ID NO: 12. Said epitope of huCEACAM1 with the amino acid sequence of SEQ ISD NO: 11 is conserved among human CEACAM1, human CEACAM3 and human CEACAM5 and, thus, the antibody of the invention or the antigen binding fragment thereof also binds specifically to huCEACAM3 and huCEACAM5.

The antibody of the invention or the antigen binding fragment thereof is particularly useful in various applications including flow cytometry, enzyme-linked immunosorbent assays (ELISA), radioimmunoassays (RIA), enzyme linked immunospot (ELISPOT), immuno-PCR western blotting, immune precipitation (IP), immuno-MRM, immuno-MALDI, immunohistochemistry (IHC), fluorescence microscopy (FluMi) and *in vivo* staining for e.g. fluorescence, radioactive and metal/metal ligation guided applications as well as contrast agent bound e.g. to microbubbles for molecular ultrasound imaging etc..

Further, it has been shown that the antibody of the invention or the antigen binding fragment thereof is capable of competitively blocking the binding of HopQ to CEACAM1. Since it is known in the art that some bacteria use HopQ-mediated binding to -huCEACAM1 during infection of a subject (see e.g. WO 2018/015468 A1), the antibody of the invention or the antigen binding fragment thereof is suitable to prevent or treat diseases like e.g. infection, in particular of bacterial infection.

The antibody of the invention also turned out to be suitable to increase epithelial and endothelial cell survival e.g. during cold storage of the cells. Thus, the antibody of the invention or the antigen binding fragment thereof can be used as additive for organ transplantation to keep the cells of the respective organ alive and functional for a prolonged period of time.

Further, it was demonstrated that the antibody of the invention or the antigen binding fragment thereof is capable of increasing proliferation and migration of epithelial cells and, thus, is suitable for supporting wound healing.

The antibody of the invention is capable of co-stimulating T-cell and B-cell proliferation and T-cell and B-cell response. Thus, the antibody of the invention or the antigen binding fragment thereof is suitable for T-cell and/or B-cell expansion *in vitro* and activation or stimulation of T-cell and/or B-cell response *in vivo.* Therefore, the antibody of the invention or the antigen binding fragment thereof can be used in treatment of various diseases associated with T-cell and/or B-cell response, activation or stimulation like e.g. immune diseases, auto-immune diseases, restoration and/or improvement of immune-response e.g. after chemotherapeutic treatment and/or as active agent in adoptive immune therapy e.g. CAR-T. The antibody of the invention or the antigen binding fragment thereof can also be used as supportive agent during vaccination of a subject or for generation of antibodies against an antigen *in vitro* and/or *in vivo* (e.g. in human or a suitable animal model).

The antibody of the invention interacts with the N domain of CEACAM1 and its binding leads to monomerization of CEACAM1 which usually appears in a non-activated state as cis-dimeric/oligomeric complex. Obviously, this monomerization opens up the opportunity for the CEACAM 1 receptor to partner with one of its co-receptors like the T and B cell receptor (TCR, BCR), the EGFR, insulin receptor, VEGFR1-3, G-CSF-R, Toll-like receptors (e.g. TLR-2 and -4). Otherwise it may go back to be cis-dimerized/oligomerized. Important to note, in the monomeric state of CEACAM1, distinct non-N domain binding anti CEACAM antibodies can bind to epitopes usually covered in the dimeric/oligomeric organization. Due to this increased accessibility of CEACAM binding site, CEACAM non-N domain antibodies can bind and trigger functions like the maturation macrophages and dendritic cells. Furthermore, the antibody of the invention is the sole one identified so far able to block the HopQ interaction of *Helicobacter pylori* with human CEACAM1, CEACAM3 and CEACAM5 and thus potentially inhibiting further pathological processes.

Preferably, the isolated antibody or antigen binding fragment of the invention binds to an epitope on huCEACAM1, especially to an epitope present in the extracellular N-domain of huCEACAM1, the epitope comprising or consisting of the amino acid residues sequence of SEQ ID NO:11.

The isolated antibody or antigen binding fragment thereof of the invention preferably comprises a heavy chain (VH) comprising or consisting of the amino acid sequence of: 
EVQLQQSGPELVKPGTSVKMSCKASGYTFTTYVMHWVQQKPGQGLDWIGFFNPYNDGTK
YNEKFKGKATLTSDKSSSTAYMELSSLTSEDSAVYYCARWAYDGSYAYWGQGTTLTVSS
(SEQ ID NO:7),
 and a light chain (VL) comprising or consisting of the amino acid sequence of:

DIQMTQSSSYLSVFLGGRVTITCKASDHINNWLAWYQQKPGNAPRLLISGATSLETGVPSRF
SGSGSGKDYTLSIISLQTEDVATYYCQQYWRTPFTFGSGTKLEIK (SEQ ID NO:8).

In a preferred embodiment, the VH region with the amino acid sequence of SEQ ID NO: 7 is encoded by a nucleic acid molecule comprising the nucleotide sequence of SEQ ID NO: 9. Alternatively or in addition, the VL region with the amino acid region of SEQ ID NO: 8 is encoded by a nucleic acid molecule comprising the nucleic acid sequence of SEQ ID NO: 10.

The isolated antibody or antigen binding fragment thereof of the invention is preferably monoclonal.

The isolated antibody or antigen binding fragment thereof of the invention preferably is:
- recombinant; or
- an IgG, IgM, IgA or an antigen binding fragment thereof; or
- a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, or a monovalent.

The isolated antibody or antigen binding fragment thereof of the invention is preferably a humanized antibody or de-immunized antibody or an antigen binding fragment thereof.

The isolated antibody or antigen binding fragment thereof of the invention can be monospecific or bi-specific.

The isolated antibody or antigen binding fragment thereof of the invention optionally is conjugated to another molecule to form an immunoconjugate, preferably the isolated antibody or antigen binding fragment thereof of the invention is conjugated to an active agent like e.g. an imaging agent, a therapeutic agent, a toxin or a radionuclide.

The present invention is further directed to a pharmaceutical composition comprising the isolated antibody or antigen binding fragment thereof of the invention and a pharmaceutically or physiologically acceptable carrier or excipient.

Further, the present invention refers to a polynucleotide molecule comprising a nucleic acid sequence encoding an isolated antibody or antigen binding fragment thereof of the present invention.

The present invention is also directed to a host cell comprising one or more polynucleotide molecule(s) encoding an isolated antibody or antigen binding fragment thereof of the invention, optionally wherein the host cell is a mammalian cell, a yeast cell, a bacterial cell, a ciliate cell, an insect cell or a plant cell. The host cell can be a eukaryotic cell, e.g., a mammalian cell, an insect cell, a yeast cell, or a prokaryotic cell, e.g., E. coli. For example, the mammalian cell can be a cultured cell or a cell line. Exemplary mammalian cells include lymphocytic cell lines (e.g., NS0), Chinese hamster ovary cells (CHO), COS and HEK-293 cells. Additionally cells include oocyte cells, and cells from a transgenic animal, e.g., mammary epithelial cell. For example, nucleic acids encoding an antibody or a modified antibody or an antigen binding fragment thereof described herein can be expressed in a transgenic animal.

Further, the present invention comprises a method of manufacturing an antibody or antigen binding fragment thereof of the invention comprising:
(a) expressing one or more polynucleotide molecule(s) encoding an isolated antibody or antigen binding fragment thereof of the invention in a cell; and
(b) purifying the antibody from the cell/cell supernatants.

The present invention also refers to kit comprising an isolated antibody or antigen binding fragment of any of the invention and instructions for use of the antibody, optionally further wherein the antibody of antigen binding fragment thereof of the invention is lyophilized.

Further, the present invention is directed to a medical use of the antibody or antigen binding fragment thereof of the invention. Preferably, the isolated antibody or antigen binding fragment of the invention or the pharmaceutical composition of the invention is intended for use in a diagnostic method of a disease or medical condition. Alternatively or in addition, the isolated antibody or antigen binding fragment of the invention or the pharmaceutical composition of the invention is provided for use in treating or preventing a disease or medical condition. In one embodiment, the antibody or antigen binding fragment of the invention or the pharmaceutical composition of the invention is used in the manufacture of a medicament for treatment or prevention of a disease or medical condition. In another embodiment, the present invention is directed to a method of treatment or prevention of a disease or medical condition, wherein a subject in need thereof is administered with an effective amount of the antibody or antigen binding fragment thereof of the invention or the pharmaceutical composition of the invention. Preferably, the disease or medical condition referred to above comprise or consist of infections by a pathogen such as a bacterium, fungus, or virus, acute and chronic inflammatory diseases, wound healing, treatment of immunodeficient or immunocompromised patients, booster for vaccinations, agent for *in vitro*/*in vivo* expansion of leukocyte subtypes like B and T lymphocytes, *in vitro*/*in vivo* maturation of dendritic cells (DCs) and macrophages, as agent supporting cell survival to be used e.g. for preservation of certain organs for transplantation (e.g. liver, kidney) and cancer immune therapy which hereby coordinates the proper functioning of the different leukocyte subtypes.

Examples of disorders that can be treated with the antibody or antigen binding fragment thereof of the invention include, but are not limited to,
- infections
- acute and chronic inflammatory diseases
- wound healing
- immunodeficiencies or immunocompromises,
- vaccinations
- leukocyte expansion and differentiation
- maturation of antigen presenting cells
- transplantation
- cancer including metastases
- diseases associated with T-cell and/or B-cell response
- immune diseases
- auto-immune diseases
- restoration and/or improvement of immune response e.g. after chemotherapeutic treatment
- adoptive immune therapy, e.g. CAR-T
- photodynamic therapy (PDT)
- photo-thermal therapy (PTT)

Unless otherwise defined herein, scientific and technical terms used in connection with the present invention have the meanings that are commonly understood by those of ordinary skill in the art. Generally, nomenclature utilized in connection with, and techniques of, cell and tissue culture, molecular biology, and protein and oligo- or polynucleotide chemistry and hybridization described herein are those well-known and commonly used in the art. Standard techniques are used for recombinant DNA, oligonucleotide synthesis, and tissue culture and transformation and transfection (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art; see e.g., Sambrook et al. Molecular Cloning: A Laboratory Manual (3rd ed., Cold Spring Harbor Laboratory Press, Cold Spring Harbor, N.Y. (2000)), and Harlow, E. and Lane, D. (1988) Antibodies: A Laboratory Manual, Cold Spring Harbor Laboratory Press, Cold Spring Harbor, NY . The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients. Furthermore, unless otherwise required by context, singular terms shall include pluralities and plural terms shall include the singular.

The instant invention is most clearly understood with reference to the following definitions, as well as additional definitions as set forth throughout the description:
As used herein, the term "antibody", "antibody peptide(s)" or "immunoglobulin" refers to single chain, two-chain, and multi-chain proteins and glycoproteins that belong to the classes of polyclonal, monoclonal, chimeric and human or humanized immunoglobulin proteins. The term "antibody" also includes synthetic and genetically engineered variants thereof.

As used herein, the term "antibody fragment" or "antigen binding fragment" of an antibody refers to a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, single chain antibodies, functional heavy chain antibodies (nanobodies), as well as any portion of an antibody having specificity toward at least one desired epitope, that competes with the intact antibody for specific binding (e.g., an isolated portion of a complementarity determining region having sufficient framework sequences so as to bind specifically to an epitope). Antigen binding fragments can be produced by recombinant techniques, or by enzymatic or chemical cleavage of an intact antibody.

As used herein, the term "human antibody" refers to an antibody that possesses a sequence that is derived from a human germ-line immunoglobulin sequence, such as antibodies derived from transgenic mice having human immunoglobulin genes (e.g., XENOMOUSE ™genetically engineered mice (Abgenix)), human phage display libraries, in cows (milk) or human B cells.

As used herein, the term "humanized antibody" refers to an antibody that is derived from a non-human antibody (e.g., murine) that retains or substantially retains the antigen-binding properties of the parent antibody but is less immunogenic in humans. Humanized as used herein is intended to include deimmunized antibodies.

The term "modified" or "recombinant" antibody, as used herein, refers to antibodies that are prepared, expressed, created or isolated by recombinant means, such as antibodies expressed using a recombinant expression vector transfected into a host cell, antibodies isolated from a recombinant, combinatorial antibody library, antibodies isolated from an animal (e.g., a mouse) that is transgenic for human immunoglobulin genes or antibodies prepared, expressed, created or isolated by any other means that involves splicing of human immunoglobulin gene sequences to other DNA sequences. Such modified antibodies include humanized, CDR grafted, chimeric, *in vitro* generated (e.g., by phage display) antibodies, and may optionally include variable or constant regions derived from human germline immunoglobulin sequences or human immunoglobulin genes or antibodies which have been prepared, expressed, created or isolated by any means that involves splicing of human immunoglobulin gene sequences to alternative immunoglobulin sequences.

The term "monospecific antibody" refers to an antibody that displays a single binding specificity and affinity for a particular target, e.g., epitope. This term includes a "monoclonal antibody" or "monoclonal antibody composition," which as used herein refer to a preparation of antibodies or fragments thereof of single molecular composition.

The term "bispecific antibody" or "bifunctional antibody" refers to an antibody that displays dual binding specificity for two epitopes, where each binding site differs and recognizes a different epitope.

It is understood that the antibodies and antigen binding fragment thereof of the invention may have additional conservative or non-essential amino acid substitutions, which do not have a substantial effect on the polypeptide functions. Whether or not a particular substitution will be tolerated, i.e., will not adversely affect desired biological properties, such as binding activity can be determined as described in Bowie, JU et al. Science 247:1306-1310 (1990). A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., asparagine, glutamine, serine, threonine, tyrosine, cysteine), nonpolar side chains (e.g., glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

A "non-essential" amino acid residue is a residue that can be altered from the wild-type sequence of the binding agent, e.g., the antibody, without abolishing or more preferably, without substantially altering a biological activity, whereas an "essential" amino acid residue results in such a change.

As used herein, the term "isolated" refers to material that is removed from its original environment (e.g., the natural environment if it is naturally occurring). For example, a naturally occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment.

As used herein, the term "vector" refers to a replicon in which another polynucleotide segment is attached, such as to bring about the replication and/or expression of the attached segment.

As used herein, the term "control sequence" refers to a polynucleotide sequence that is necessary to effect the expression of a coding sequence to which it is ligated. The nature of such control sequences differs depending upon the host organism. In prokaryotes, such control sequences generally include a promoter, a ribosomal binding site and terminators and, in some instances, enhancers. The term "control sequence" thus is intended to include at a minimum all components whose presence is necessary for expression, and also may include additional components whose presence is advantageous, for example, leader sequences.

As used herein, the term "purified product" refers to a preparation of the product which has been isolated from the cellular constituents with which the product is normally associated and from other types of cells that may be present in the sample of interest.

As used herein, the term "epitope" refers to any protein determinate capable of binding specifically to an antibody or T-cell receptors. Epitopic determinants usually consist of chemically active surface groupings of molecules such as amino acids or sugar side chains and usually have specific three dimensional structural characteristics, as well as specific charge characteristics.

As used herein, "isotype" refers to the antibody class (e.g., IgM; IgA or IgG) that is encoded by heavy chain constant region genes.

The term "agent" or "active agent" is used herein to denote a radionuclide, a chemical compound, a mixture of chemical compounds, a biological macromolecule, or an extract made from biological materials. The term "agent" or "active agent" comprises e.g. imaging agents, therapeutic agents, toxins and radionuclides.

As used herein, the terms "label" or "imaging agent" refers to a detectable marker that can be incorporated, e.g., by incorporation of a radiolabelled amino acid or attachment to a polypeptide of biotinyl moieties that can be detected by marked avidin (e.g., streptavidin containing a fluorescent marker or enzymatic activity that can be detected by optical or calorimetric methods). Useful detectable agents or imaging agents with which an antibody or an antibody portion of the invention may be labelled include fluorescent, infrared, x-ray, ultrasound compounds or coupled to e.g. air or gas filled material, metal and metal ligations (e.g. for photothermal therapy), various enzymes, prosthetic groups, luminescent materials, bioluminescent materials, fluorescent emitting metal atoms, e.g., europium (Eu), and other anthanides, and radioactive materials (described above). Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, b-galactosidase, acetylcholinesterase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a coloured reaction product, which is detectable. An antibody may also be derivatized with a prosthetic group (e.g., streptavidin/biotin and avidin/biotin). For example, an antibody may be derivatized with biotin, and detected through indirect measurement of avidin or streptavidin binding. Examples of suitable fluorescent materials include umbelliferon, fluorescein, fluorescein isothiocyanate, rhodamine, dichlorotriazinylamine fluorescein, dansyl chloride or phycoerythrin; an example of a luminescent material includes luminol; and examples of bioluminescent materials include luciferase, luciferin, and aequorin. In certain situations, the label or imaging agent can also be therapeutic. Various methods of labelling polypeptides and glycoproteins are known in the art and may be used. Examples of labels or imaging agents for polypeptides like e.g. the antibody or the antigen binding fragment thereof of the invention include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent labels (e.g., FITC, rhodamine, lanthanide phosphors), enzymatic labels (e.g., horseradish peroxidase, beta-galactosidase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags), metal and metal ligations. In some embodiments, labels or imaging agents are attached by spacer arms of various lengths to reduce potential steric hindrance.

Alternatively or in addition, the antibody or antigen binding fragment thereof of the invention can be conjugated to a therapeutic agent. Examples of therapeutic agents include, but are not limited to, cytochalasin B, gramicidin D, ethidium bromide, emetine, etoposide, tenoposide, colchicin, dihydroxy anthracin dione, mitoxantrone, 1-dehydrotestosterone, glucocorticoids, procaine, tetracaine, lidocaine, propranolol, antimetabolites (e.g., methotrexate, 6-mercaptopurine, 6-thioguanine, cytarabine, 5-fluorouracil decarbazine), alkylating agents (e.g., mechlorethamine, thioepa chlorambucil, CC-1065(see US Patent Nos. 5,475,092, 5,585,499, 5,846,545), melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, mitomycin, puromycin anthramycin (AMC)), duocarmycin and analogs or derivatives thereof, and anti-mitotic agents (e.g., vincristine, vinblastine, taxol, auristatins (e.g., auristatin E) and maytansinoids, and analogs or homologs thereof.

Alternatively or in addition, the antibody or antigen binding fragment thereof of the invention can also be coupled a radioactive isotope or radionuclide. Radioactive isotopes can be used in diagnostic or therapeutic applications. Radioactive isotopes that can be coupled to the antibodies include, but are not limited to α-, β-, or γ-emitters, or β- and γ-emitters. Such radioactive isotopes include, but are not limited to iodine (¹³¹I or ¹²⁵I), yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (^{99m}Tc), phosphorus (³²P), rhodium (⁸⁸Rh), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), or gallium (⁶⁷Ga). Radioisotopes useful as therapeutic agents include yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), and rhodium (¹⁸⁸Rh). Radioisotopes useful as labels, e.g., for use in diagnostics, include iodine (¹³¹I or ¹²⁵I), indium (¹¹¹In), technetium (^{99m}Tc), phosphorus (³²P), carbon (¹⁴C), and tritium (³H), or one or more of the therapeutic isotopes listed above.

As used herein, "specific binding" "bind(s) specifically" or "binding specificity" refers to the property of the antibody to: (1) to bind to huCEACAM1, 3 and 5, e.g., human CEACAM1 protein, with an affinity of at least 10⁻⁷ M, and (2) preferentially bind to huCEACAM1, 3 and 5, e.g., human CEACAM1 protein, with an affinity that is at least two-fold, 50-fold, 100-fold, 1000-fold, or more greater than its affinity for binding to a non-specific antigen (e.g., BSA, casein) other than huCEACAM1, huCEACAM3 and huCEACAM5.

As used herein, the term "treat" or "treatment" is defined as the application or administration of an antibody or antigen binding fragment thereof of the invention to a subject, e.g., a subject in need thereof or a patient, or application or administration to an isolated tissue or cell from a subject, e.g., a patient, which is returned to the subject. The antibody or antigen binding fragment thereof of the invention can be administered alone or in combination with a second agent. The treatment can be to cure, heal, alleviate, relieve, alter, remedy, ameliorate, palliate, improve or affect the disorder, disease or medical condition, the symptoms of the disorder or the predisposition toward the disorder.

Disorders or medical conditions that can be treated with the antibody or antigen binding fragment thereof of the invention comprise infections by pathogens, acute and chronic inflammatory diseases, wound healing problems, immunodeficiencies or immunocompromises, non-responders in vaccinations, insufficient leukocyte expansion and differentiation upon stimulation, reduced maturation of antigen presenting cells, apoptotic processes in transplant organs, and insufficient immune response to malignant transformed cells and their metastasis.

Examples of potential applications include but are not limited to,
- infections
- acute and chronic inflammatory diseases
- immunodeficiencies or immunocompromises,
- vaccinations,
- leukocyte expansion and differentiation
- maturation of antigen presenting cells
- transplantation
- cancer/cancer metastasis
- photothermal therapy
- adopted T cell transfer
- wound healing
- leukocyte expansion and differentiation
- diseases associated with T-cell and/or B-cell response
- immune diseases
- auto-immune diseases
- restoration and/or improvement of immune response e.g. after chemotherapeutic treatment
- adoptive immune therapy, e.g. CAR-T cell therapy
- photodynamic therapy (PDT)
- photo-thermal therapy (PTT)

As used herein, an amount of an antibody or antigen binding fragment thereof of the invention "effective" or "sufficient" to treat a disorder, or a "therapeutically effective amount" or "therapeutically sufficient amount "refers to an amount of the antibody or antigen binding fragment thereof of the invention which is effective, upon single or multiple dose administration to a subject, in treating a cell, or in prolonging, curing, alleviating, relieving or improving a subject with a disorder as described herein beyond that expected in the absence of such treatment.

As used herein, "huCEACAM1" refers to human CEACAM1 with the amino acid sequence of SEQ ID NO: 12. The huCEACAM1 protein comprises 526 amino acids, and is described in Genbank accession no.: P13688.

The antibodies or antigen binding fragments thereof of the invention interact with, e.g., bind to the extracellular N domain of huCEACAM1 located at about amino acids 59 to 68 of huCEACAM1 within SEQ ID NO: 12. In one embodiment, the antibody or antigen binding fragment thereof of the invention binds all or part of the epitope on huCEACAM1 comprising amino acids of the polypeptide of SEQ ID NO: 11. The antibody of the invention can inhibit, e.g., competitively inhibit, the binding of HopQ, a protein of *Helicobacter pylori* specifically binding to huCEACAM1; as described e.g. in WO 2018/015468 A1.

### Antibodies:

The antibody structural unit is a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 120 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)). The variable regions of each light chain (VL) and heavy chain (VH) pair form the antibody binding site. Preferred isotypes for the antibodies of the invention are IgG immunoglobulins, which are classified into four subclasses, IgG1, IgG2, IgG3 and IgG4, having different gamma heavy chains. Most therapeutic antibodies are human, chimeric, or humanized antibodies of the IgG1 type.

The variable regions of each heavy and light chain pair form the antigen binding site. Thus, an intact IgG antibody has two binding sites which are the same. However, bifunctional or bispecific antibodies are artificial hybrid constructs which have two different heavy/light chain pairs, resulting in two different binding sites.

The chains all exhibit the same general structure of relatively conserved framework regions (FR) joined by three hyper variable regions, also called complementarity determining regions or CDRs. The CDRs from the two chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each domain is in accordance with the definitions of the IMTG unique Lefranc numbering, Lefranc,M.P.,The Immunologist, 7,132-136(1999), homepage IMTG® http://www.imtg.org. As used herein, CDRs are referred to for each of the heavy (HCDR1, HCDR2, HCDR3) and light (LCDR1, LCDR2, LCDR3) chains.

The antibodies of the present invention can be polyclonal antibodies, monoclonal antibodies, chimeric antibodies (see U.S. Pat. No. 6,020,153) or human or humanized antibodies or antibody fragments or derivatives thereof. Synthetic and genetically engineered variants (see U.S. Pat. No. 6,331,415) of any of the foregoing are also contemplated by the present invention. Monoclonal antibodies can be produced by a variety of techniques, including conventional murine monoclonal antibody methodology e.g., the standard somatic cell hybridization technique of Kohler and Milstein, Nature 256: 495 (1975). See generally, Harlow, E. and Lane, D. (1988) Antibodies: A Laboratory Manual, Cold Spring Harbor Laboratory Press, Cold Spring Harbor, NY . Preferably, the antibodies of the present invention are human or humanized antibodies. The advantage of human or humanized antibodies is that they potentially decrease or eliminate the immunogenicity of the antibody in a host recipient, thereby permitting an increase in the bioavailability and a reduction in the possibility of adverse immune reaction, thus potentially enabling multiple antibody administrations.

Modified antibodies include humanized, chimeric or CDR-grafted antibodies. Human anti-mouse antibody (HAMA) responses have led to development of chimeric or otherwise humanized antibodies. While chimeric antibodies have a human constant region and a murine variable region, it is expected that certain human anti-chimeric antibody (HACA) responses will be observed, particularly in chronic or multi-dose utilizations of the antibody. The presence of such murine or rat derived proteins can lead to the rapid clearance of the antibodies or can lead to the generation of an immune response against the antibody by a patient. In order to avoid the utilization of murine or rat derived antibodies, humanized antibodies where sequences are introduced to an antibody sequence to make it closer to human antibody sequence, or fully human antibodies generated by the introduction of human antibody function into a rodent have been developed so that the rodent would produce antibodies having fully human sequences. Human antibodies avoid certain of the problems associated with antibodies that possess murine, rabbit, or rat variable and/or constant regions.

### Human Antibodies:

Fully human antibodies are expected to minimize the immunogenic and allergic responses intrinsic to mouse or mouse-derivatized mAbs and thus to increase the efficacy and safety of the administered antibodies. The use of fully human antibodies can be expected to provide a substantial advantage in the treatment of chronic and recurring human diseases, such as inflammation, autoimmunity, and cancer, which require repeated antibody administrations. Also, human antibodies can be produced using genetically engineered strains of animals in which the antibody gene expression of the animal is suppressed and functionally replaced with human antibody gene expression.

Methods for making humanized and human antibodies are known in the art. One method for making human antibodies employs the use of transgenic animals, such as a transgenic mouse. These transgenic animals contain a substantial portion of the human antibody producing genome inserted into their own genome and the animal's own endogenous antibody production is rendered deficient in the production of antibodies. Methods for making such transgenic animals are known in the art. Such transgenic animals can be made using XENOMOUSE™ technology or by using a "minilocus" approach. Methods for making XENOMICE™ are described in U.S. Pat. Nos. 6,162,963, 6,150,584, 6,114,598 and 6,075,181. Methods for making transgenic animals using the "minilocus" approach are described in U.S. Pat. Nos. 5,545,807, 5,545,806 and 5,625,825; also see International Publication No. WO93/12227.

Using the XENOMOUSE™ technology, human antibodies can be obtained by immunizing a XENOMOUSE™ mouse (Abgenix, Fremont, Calif.) with an antigen of interest. The lymphatic cells (such as B-cells) are recovered from the mice that express antibodies. These recovered cells can be fused with myeloid-type cell line to prepare immortal hybridoma cell lines, using standard methodology. These hybridoma cell lines can be screened and selected to identify hybridoma cell lines that produce antibodies specific to the antigen of interest. Alternatively, the antibodies can be expressed in cell lines other than hybridoma cell lines. More specifically, sequences encoding particular antibodies can be cloned from cells producing the antibodies and used for transformation of a suitable mammalian host cell. In a preferred method, spleen and/or lymph node lymphocytes from immunized mice are isolated from the mice and plated in plaque assays as known in the art. Briefly, cells are plated in agar with sheep red blood cells, coated with antigen and cells secreting mAb against the antigen would fix complement and lyse the red blood cells immediately surrounding the mAb producing cells. Cells within the cleared plaques are lifted for sequencing of the immunoglobulin sequences and subcloning into expression vectors. Supernatants from transiently transfected cells containing antigen-specific mAb were subsequently screened by ELISA and for binding to cells by flow cytometry. The variable sequences, or a portion thereof of the produced human antibodies comprising complementarity determining regions which bind particular epitopes may be utilized for production of modified antibodies. For example, the variable regions of the produced antibodies may be spliced into an expression cassette for ease of transfer of constructs, increased expression of constructs, and/or incorporation of constructs into vectors capable of expression of full length antibodies.

### Chimerisation, Humanization and Display Technologies:

As discussed above, there are advantages to producing antibodies with reduced immunogenicity. To a degree, this can be accomplished in connection with techniques of humanization and display techniques using appropriate libraries. It will be appreciated that murine antibodies or antibodies from other species can be humanized or primatized using techniques well known in the art. The antibody of interest may be engineered by recombinant DNA techniques to substitute the CH1, CH2, CH3, hinge domains, and/or the framework domain with the corresponding human sequence (see e.g. WO 92/02190 A1, US 5,530,101, US 5,585,089, US 5,693,761, US 5,693,792, US 5,714,350, and US 5,777,085). Also, the use of Ig cDNA for construction of chimeric immunoglobulin genes is known in the art. mRNA is isolated from a hybridoma or other cell producing the antibody and used to produce cDNA: The cDNA of interest may be amplified by the polymerase chain reaction using specific primers (see e.g. US 4,683,195 and US 4,683,202). Alternatively, a library is made and screened to isolate the sequence of interest. The DNA sequence encoding the variable region of the antibody is then fused to human constant region sequences. The sequences of human constant regions genes may be found in Kabat et al. (1991) Sequences of Proteins of Immunological Interest, N.I.H. publication no. 91-3242. Human C region genes are readily available from known clones. The choice of isotype will be guided by the desired effector functions, such as complement fixation, or activity in antibody-dependent cellular cytotoxicity. Isotypes can be IgG1, IgG2, IgG3 or IgG4. Preferred isotypes for antibodies of the invention are IgG1 and IgG2. Either of the human light chain constant regions, kappa or lambda, may be used. The chimeric, humanized antibody is then expressed by conventional methods.

Humanized antibodies can also be made using a CDR-grafted approach. Techniques of generation of such humanized antibodies are well known in the art. Generally, humanized antibodies are produced by obtaining nucleic acid sequences that encode the variable heavy and variable light sequences of an antibody that binds to the antigen of interest, identifying the complementary determining region or "CDR" in the variable heavy and variable light sequences and grafting the CDR nucleic acid sequences on to human framework nucleic acid sequences (see, for example, US 4,816,567 and US 5,225,539). The location of the CDRs and framework residues can be determined using known technology. The human framework that is selected is one that is suitable for *in vivo* administration, meaning that it does not exhibit immunogenicity. For example, such a determination can be made by prior experience with *in vivo* usage of such antibodies and studies of amino acid similarities.

Once the CDRs and FRs of the cloned antibody that are to be humanized are identified, the amino acid sequences encoding the CDRs are identified and the corresponding nucleic acid sequences grafted on to selected human FRs. This can be done using known primers and linkers, the selection of which are known in the art. Alternatively, the entire variable regions can be chemically synthesized without the need of a template. All of the CDRs of a particular human antibody may be replaced with at least a portion of a non-human CDR or only some of the CDRs may be replaced with non-human CDRs. It is only necessary to replace the number of CDRs required for binding of the humanized antibody to a predetermined antigen. After the CDRs are grafted onto selected human FRs, the resulting "humanized" variable heavy and variable light sequences are expressed to produce a humanized Fv or humanized antibody that binds to the antigen of interest. Typically, the humanized variable heavy and light sequences are expressed as a fusion protein with human constant domain sequences so an intact antibody that binds to the antigen of interest is obtained. However, a humanized Fv antibody can be produced that does not contain the constant sequences.

Also within the scope of the invention are humanized antibodies in which specific amino acids have been substituted, deleted or added. In particular, humanized antibodies have amino acid substitutions in the framework region, such as to improve binding to the antigen. For example, a selected, small number of acceptor framework residues of the humanized immunoglobulin chain can be replaced by the corresponding donor amino acids. Locations of the substitutions include amino acid residues adjacent to the CDR, or which are capable of interacting with a CDR (see e.g. US 5,585,089). The acceptor framework can be a mature human antibody framework sequence or a consensus sequence. As used herein, the term "consensus sequence" refers to the sequence formed from the most frequently in a region among related family members.

Other techniques for humanizing antibodies are described in EP 519596 A1. In addition, the invention covers also the humanization by guided selection and chain shuffling, where the murine variable domains are sequentially and functionally replaced by complementary human region displayed on filamentous phages.

The antibody or antigen binding fragment thereof of the invention includes other humanized antibodies which may also be modified by specific deletion of human T cell epitopes or "deimmunization" by the methods disclosed in WO 98/52976 A1 and WO 00/34317 A1. Briefly, the murine heavy and light chain variable regions of an antibody can be analysed for peptides that bind to MHC Class II; these peptides represent potential T-cell epitopes. For detection of potential T-cell epitopes, a computer modelling approach termed "peptide threading" can be applied, and in addition a database of human MHC class II binding peptides can be searched for motifs present in the murine VH and VL sequences, as described in WO 98/52976 A1 and WO 00/34317 A1. These motifs bind to any of the 18 major MHC class II DR allotypes, and thus constitute potential T cell epitopes. Potential T-cell epitopes detected can be eliminated by substituting small numbers of amino acid residues in the variable regions, or preferably, by single amino acid substitutions. As far as possible conservative substitutions are made, often but not exclusively, an amino acid common at this position in human germline antibody sequences may be used. The V BASE directory provides a comprehensive directory of human immunoglobulin variable region sequences (compiled by Tomlinson, I.A. et al. MRC Centre for Protein Engineering, Cambridge, UK). After the deimmunized VH and VL of an antibody are constructed by mutagenesis of the murine VH and VL genes, the mutagenized variable sequence can, optionally, be fused to a human constant region, e.g., human IgG1 or κ constant regions.

Antibodies of the invention that are not intact antibodies are also useful in this invention. Such antibodies may be derived from any of the antibodies described above. For example, antigen-binding fragments, as well as full-length monomeric, dimeric or trimeric polypeptides derived from the above-described antibodies are themselves useful. Useful antibody homologs of this type include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment, which consists of a VH domain; (vii) a single domain functional heavy chain antibody, which consists of a VHH domain (known as a nanobody).

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules, known as single chain Fv (scFv). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies. Antibody fragments, such as Fv, F(ab')2 and Fab may be prepared by cleavage of the intact protein, e.g. by protease or chemical cleavage.

In one approach, consensus sequences encoding the heavy and light chain J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, cosmids, YACs, EBV derived episomes, and the like. A convenient vector is one that encodes a functionally complete human CH or CL immunoglobulin sequence, with appropriate restriction sites engineered so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody may be joined to any strong promoter, Examples of suitable vectors that can be used include those that are suitable for mammalian hosts and based on viral replication systems, such as simian virus 40 (SV40), Rous sarcoma virus (RSV), adenovirus 2, bovine papilloma virus (BPV), papovavirus BK mutant (BKV), or mouse and human cytomegalovirus (CMV), and moloney murine leukemia virus (MMLV), native Ig promoters, etc.

Expression in eukaryotic host cells is useful because such cells are more likely than prokaryotic cells to assemble and secrete a properly folded and immunologically active antibody. However, any antibody produced that is inactive due to improper folding may be renaturable according to well-known methods. It is possible that the host cells will produce portions of intact antibodies, such as light chain dimers or heavy chain dimers, which also are antibody homologs according to the present invention.

Further, human antibodies or antibodies from other species can be generated through display-type technologies, including, without limitation, phage display, retroviral display, ribosomal display, and other techniques, using techniques well known in the art and the resulting molecules can be subjected to additional maturation, such as affinity maturation, as such techniques are well known in the art. If display technologies are utilized to produce antibodies that are not human, such antibodies can be humanized as described above.

It will be appreciated that antibodies that are generated need not initially possess a particular desired isotype but, rather, the antibody as generated can possess any isotype and the antibody can be isotype switched thereafter using conventional techniques that are well known in the art. Such techniques include the use of direct recombinant techniques (see e.g. US 4,816,397), cell-cell fusion techniques (see e.g. US 5,916,771), among others. In the cell-cell fusion technique, a myeloma or other cell line is prepared that possesses a heavy chain with any desired isotype and another myeloma or other cell line is prepared that possesses the light chain. Such cells can, thereafter, be fused and a cell line expressing an intact antibody can be isolated.

In connection with bispecific antibodies, bispecific antibodies can be generated that comprise (i) two antibodies one with a specificity to huCEACAM1 and another to a second molecule that are conjugated together, (ii) a single antibody that has one chain specific to huCEACAM1 and a second chain specific to a second molecule, or (iii) a single chain antibody that has specificity to huCEACAM1 and the other molecule. Such bispecific antibodies can be generated using techniques that are well known. For example, bispecific antibodies may be produced by crosslinking two or more antibodies (of the same type or of different types). Suitable crosslinkers include those that are heterobifunctional, having two distinctly reactive groups separated by an appropriate spacer (e.g., m-maleimidobenzoyl-N-hydroxysuccinimide ester) or homobifunctional (e.g., disuccinimidyl suberate).

### Nucleic acid and Polypeptides:

In another embodiment, the present invention relates to polynucleotide and polypeptide sequences that encode for the antibodies or fragments thereof described herein. Such polynucleotides encode for both the variable and constant regions of each of the heavy and light chains, although other combinations are also contemplated by the present invention in accordance with the compositions described herein. The present invention also contemplates oligonucleotide fragments derived from the disclosed polynucleotides and nucleic acid sequences complementary to these polynucleotides.

The polynucleotides can be in the form of RNA or DNA. Polynucleotides in the form of DNA, cDNA, genomic DNA, nucleic acid analogues and synthetic DNA are within the scope of the present invention. The DNA may be double-stranded or single-stranded, and if single stranded, may be the coding (sense) strand or non-coding (anti-sense) strand. The coding sequence that encodes the polypeptide may be identical to the coding sequence provided herein or may be a different coding sequence which coding sequence, as a result of the redundancy or degeneracy of the genetic code, encodes the same polypeptide as the DNA provided herein.

The provided polynucleotides encode at least one heavy chain variable region and at least one light chain variable region of the present invention. Examples of such polynucleotides are shown in SEQ ID NOS: 9 and 10 as well as fragments, complements and degenerate codon equivalents thereof. For example, the polynucleotide with SEQ ID NO: 9 encodes for the heavy chain variable region VH with SEQ ID NO: 7 comprising HCDR1 with SEQ ID NO: 1, HCDR2 with SEQ ID NO: 2 and HCDR3 with SEQ ID NO: 3; and the polynucleotide with SEQ ID NO:10 encodes for the light chain variable region VL with SEQ ID NO: 8 comprising LCDR1 with SEQ ID NO: 4, LCDR2 with SEQ ID NO: 5 and LCDR3 with SEQ ID NO: 6.

The present invention also includes variant polynucleotides containing modifications such as polynucleotide deletions, substitutions or additions, and any polypeptide modification resulting from the variant polynucleotide sequence. A polynucleotide of the present invention may also have a coding sequence that is a variant of the coding sequence provided herein.

The present invention further disclose polypeptides that comprise or form part of the antibodies or antigen binding fragment thereof of the present invention as well as fragments, analogues and derivatives of such polypeptides. The polypeptides may be recombinant polypeptides, naturally produced polypeptides or synthetic polypeptides. The fragment, derivative or analogues of the polypeptides may be one in which one or more of the amino acid residues is substituted with a conserved or non-conserved amino acid residue (preferably a conserved amino acid residue) and such substituted amino acid residue may or may not be one encoded by the genetic code; or it may be one in which one or more of the amino acid residues includes a substituent group; or it may be one in which the polypeptide is fused with another compound, such as a compound to increase the half-life of the polypeptide (for example, polyethylene glycol); or it may be one in which the additional amino acids are fused to the polypeptide, such as a leader or secretory sequence or a sequence that is employed for purification of the polypeptide or a proprotein sequence. In various aspects, the polypeptides may be partially purified.

A polypeptide may have an amino acid sequence that is identical to that of the antibodies or antigen binding fragment thereof described herein or that is different by minor variations due to one or more amino acid substitutions. The variation may be a "conservative change" typically in the range of about 1 to 5 amino acids, wherein the substituted amino acid has similar structural or chemical properties, e.g., replacement of leucine with isoleucine or threonine with serine; replacement of lysine with arginine or histidine. In contrast, variations may include non-conservative changes, e.g., replacement of a glycine with a tryptophan. Similar minor variations may also include amino acid deletions or insertions or both. Guidance in determining which and how many amino acid residues may be substituted, inserted, or deleted without changing biological or immunological activity may be found using computer programs well known in the art, for example DNASTAR software (DNASTAR, Inc., Madison, Wis.).

The provided polypeptides encode at least one heavy chain variable region or at least one light chain variable region of the antibody or antigen binding fragment thereof of the present invention. The provided polypeptides can encode at least one heavy chain variable region and one light chain variable region of the antibodies of the present invention. Examples of such polypeptides are those having the amino acid sequences shown in SEQ ID NOS: 1, 2, 3, 4, 5, 6, 7, and 8, and fragments thereof. Preferably, the heavy variable region VH has the amino acid sequence shown in SEQ ID NO:7 and the light variable region VL has the amino acid sequence shown in SEQ ID NO:8. The heavy chain CDR sequences of the antibody or antigen binding fragment thereof of the invention have the amino acid sequence shown in SEQ ID NO:1 (HCDR1), SEQ ID NO:2 (HCDR2) and SEQ ID NO:3 (HCDR3); and the light chain CDRs have the amino acid sequence shown in SEQ ID NO:4 (LCDR1); GAT or GATX as in SEQ ID NO:5 (LCDR2); and SEQ ID NO:6 (LCDR3).

The present invention also provides vectors that include the polynucleotides of the present invention, host cells which are genetically engineered with vectors of the present invention and the production of the antibodies of the present invention by recombinant techniques.

The appropriate DNA sequence may be inserted into the vector by a variety of procedures. In general, the DNA sequence is inserted into appropriate restriction endonuclease sites by procedures known in the art. The polynucleotide sequence in the expression vector is operatively linked to an appropriate expression control sequence (i.e. promoter) to direct mRNA synthesis. Examples of such promoters include, but are not limited to, the LTR or the SV40 promoter, the E. coli lac or trp, the phage lambda PL promoter and other promoters known to control expression of genes in prokaryotic or eukaryotic cells or their viruses. The expression vector also contains a ribosome binding site for translation initiation and a transcription terminator. The vector may also include appropriate sequences for amplifying expression. For example, the vector can contain enhancers, which are transcription-stimulating DNA sequences of viral origin, such as those derived from simian virus such as SV40, polyoma virus, cytomegalovirus, bovine papilloma virus or Moloney sarcoma virus, or genomic, origin. The vector preferably also contains an origin of replication. The vector can be constructed to contain an exogenous origin of replication or, such an origin of replication can be derived from SV40 or another viral source, or by the host cell chromosomal replication mechanism.

In addition, the vectors optionally contain a marker gene for selection of transfected host cells such as dihydrofolate reductase or antibiotics, such as neomycin, G418 (geneticin, a neomycin-derivative) or hygromycin, or genes which complement a genetic lesion of the host cells such as the absence of thymidine kinase, hypoxanthine phosphoribosyl transferase, dihydrofolate reductase, glutamine synthetase etc.

In order to obtain the antibodies of the present invention, one or more polynucleotide sequences that encode for the light and heavy chain variable regions and light and heavy chain constant regions of the antibodies of the present invention should be incorporated into a vector. Polynucleotide sequences encoding the light and heavy chains of the antibodies of the present invention can be incorporated into one or multiple vectors and then incorporated into the host cells.

As will be appreciated, antibodies in accordance with the present invention can be expressed in cell lines other than hybridoma cell lines. Sequences encoding the cDNAs or genomic clones for the particular antibodies can be used for a suitable mammalian or non-mammalian host cells. Transformation can be by any known method for introducing polynucleotides into a host cell, including, for example packaging the polynucleotide in a virus (or into a viral vector) and transducing a host cell with the virus (or vector) or by transfection procedures known in the art, for introducing heterologous polynucleotides into mammalian cells, e.g., dextran-mediated transfection, calcium phosphate precipitation, polybrene mediated transfection, protoplast fusion, electroporation, encapsulation of the polynucleotide(s) into liposomes and direct microinjection of the DNA molecule. The transformation procedure used depends upon the host to be transformed. Methods for introduction of heterologous polynucleotides into mammalian cells are well known in the art and include, but are not limited to, dextran-mediated transfection, calcium phosphate precipitation, polybrene mediated transfection, protoplast fusion, electroporation, particle bombardment, encapsulation of the polynucleotide(s) in liposomes, peptide conjugates, dendrimers, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, NSO cells, HeLa cells, baby hamster kidney (BHK) cells, monkey kidney cells (COS), human hepatocellular carcinoma cells (e.g., Hep G2), HEK-293 and a number of other cell lines. Non-mammalian cells including but not limited to bacterial, yeast, insect, and plants can also be used to express recombinant antibodies. Site directed mutagenesis of the antibody CH2 domain to eliminate glycosylation may be preferred in order to prevent changes in either the immunogenicity, pharmacokinetic, and/or effector functions resulting from non-human glycosylation. The expression methods are selected by determining which system generates the highest expression levels and produce antibodies with constitutive antigen binding properties.

Further, expression of antibodies of the invention (or other moieties therefrom) from production cell lines can be enhanced using a number of known techniques. For example, the glutamine synthetase and DHFR gene expression systems are common approaches for enhancing expression under certain conditions. High expressing cell clones can be identified using conventional techniques, such as limited dilution cloning, Microdrop technology, or any other methods known in the art. The GS system is discussed in whole or part in connection with European Patent Nos. EP 0 216 846, EP 0 256 055, EP 0 338 841 and EP 0 323 997.

In an exemplary system for recombinant expression of a modified antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to enhancer/promoter regulatory elements (e.g., derived from SV40, CMV, adenovirus and the like, such as a CMV enhancer/AdMLP promoter regulatory element or an SV40 enhancer/AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Antibodies of the invention can also be produced transgenically through the generation of a mammal or plant that is transgenic for the immunoglobulin heavy and light chain sequences of interest and production of the antibody in a recoverable form therefrom. In connection with the transgenic production in mammals, antibodies can be produced in, and recovered from, the milk of goats, cows, or other mammals; see, e.g., US 5,827,690, US 5,756,687, US 5,750,172, and US 5,741,957.

### Conjugates:

As used herein, "immunoconjugate" comprises an antibody or antigen binding fragment of the invention, which is conjugated to a moiety comprising an agent or active agent or modified as described in more detail herein.

As used herein, a "moiety" of an immunoconjugate is intended to refer to a component of the conjugate (e.g., an immunoglobulin moiety (i.e., an antibody or antigen binding fragment or derivative thereof), a therapeutic moiety, an imaging moiety). An antibody or an antigen binding fragment thereof of the invention may be conjugated to another molecular entity, e.g., a cytotoxic or cytostatic agent, e.g., a therapeutic agent, a drug, a compound emitting radiation, molecules of plant, fungal, or bacterial origin, or a biological protein (e.g., a protein toxin) or particle (e.g., a recombinant viral particle, e.g., via a viral coat protein); a detectable agent; a pharmaceutical agent; and/or a protein or peptide that can mediate association of the antibody or antibody portion with another molecule (such as a streptavidin core region or a polyhistidine tag). For example, an antibody or antibody portion of the invention can be functionally linked by any suitable method (e.g., chemical coupling, genetic fusion, noncovalent association or otherwise) to one or more other molecular entities. Examples of linkers capable of being used to couple an immunotoxin to an antibody or antibody portion of the invention include, for example, N-succinimidyl 3-(2-pyridyldithio)proprionate (also known as N-succinimidyl 4-(2-pyridyldithio)pentanoate or SPP); 4-succinimidyl-oxycarbonyl-a-(2-pyridyldithio)-toluene (SMPT); N-succinimidyl-3-(2-pyridyldithio)butyrate (SDPB); 2-iminothiolane; S-acetylsuccinic anhydride; disulfide benzyl carbamate; carbonate; hydrazone linkers; N-(a-Maleimidoacetoxy)succinimide ester; N-[4-(p-Azidosalicylamido)butyl]-3'-(2'-pyridyldithio)propionamide (AMAS); N-[β-Maleimidopropyloxy]succinimide ester (BMPS); [N-e-Maleimidocaproyloxy]succinimide ester (EMCS); N-[g-Maleimidobutyryloxy]succinimide ester (GMBS); Succinimidyl-4-[N-Maleimidomethyl]cyclohexane-1-carboxy-[6-amidocaproate] (LC-SMCC); Succinimidyl 6-(3-[2-pyridyldithio]-propionamido)hexanoate (LC-SPDP); m-Maleimidobenzoyl-N-hydroxysuccinimide ester (MBS); N-Succinimidyl[4-iodoacetyl]aminobenzoate (SIAB); Succinimidyl 4-[N-maleimidometbyl]cyclohexane-1-carboxylate (SMCC); N-Succinimidyl 3-[2-pyridyldithio]-propionamido (SPDP); [N-e-Maleimidocaproyloxy]sulfosuccinimide ester (Sulfo-EMCS); N-[g-Maleimidobutyryloxy]sulfosuccinimide ester (Sulfo-GMBS); 4-Sulfosuccinimidyl-6-methyl-α-(2-pyridyldithio)toluamido]hexanoate) (Sulfo-LC-SMPT); Sulfosuccinimidyl 6-(3'-[2-pyridyldithio]-propionamido)hexanoate (Sulfo-LC-SPDP); m-Maleimidobenzoyl-N-hydroxysulfosuccinimide ester (Sulfo-MBS); N-Sulfosuccinimidyl[4-iodoacetyl]aminobenzoate (Sulfo-SIAB); Sulfosuccinimidyl 4-[N-maleirnidomethyl]cyclohexane-1-carboxylate (Sulfo-SMCC); Sulfosuccinimidyl 4-[p-maleimidophenyl]butyrate (Sulfo-SMPB); EGS; DST; DOTA; DTPA; and thiourea linkers.

In connection with immunoconjugates, the provided antibodies or antigen binding fragments thereof of the invention can be modified to act as immunoconjugates utilizing techniques that are well known in the art; see e.g. US 5,194,594. In connection with the preparation of radiolabeled antibodies, such modified antibodies can also be readily prepared utilizing techniques that are well known in the art; see e.g. US 4,681,581, US 4,735,210, US 5,101,827, US 5,102,990, US 5,648,471, and US 5,697,902.

As discussed, the antibody or antigen binding fragment thereof of the invention can be conjugated to a therapeutic agent. Examples of therapeutic agents include, but are not limited to, cytochalasin B, gramicidin D, ethidium bromide, emetine, etoposide, tenoposide, colchicin, dihydroxy anthracin dione, mitoxantrone, 1-dehydrotestosterone, glucocorticoids, procaine, tetracaine, lidocaine, propranolol, antimetabolites (e.g., methotrexate, 6-mercaptopurine, 6-thioguanine, cytarabine, 5-fluorouracil decarbazine), alkylating agents (e.g., mechlorethamine, thioepa chlorambucil, CC-1065(see e.g. US 5,475,092, US 5,585,499, US 5,846,545), melphalan, carmustine (BSNU) and lomustine (CCNU), cyclothosphamide, busulfan, dibromomannitol, streptozotocin, granulysin (GNLY), mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, mitomycin, puromycin anthramycin (AMC)), duocarmycin and analogs or derivatives thereof, and anti-mitotic agents (e.g., vincristine, vinblastine, taxol, auristatins (e.g., auristatin E) and maytansinoids, and analogs or homologs thereof.

The conjugates of the invention can be used for modifying a given biological response. The therapeutic agent is not to be construed as limited to classical chemical therapeutic agents. For example, the therapeutic agent may be a protein or polypeptide possessing a desired biological activity. Such proteins may include, for example, a toxin such as abrin, ricin A, pseudomonas exotoxin, gelonin, diphtheria toxin, or a component thereof (e.g., a component of pseudomonas exotoxin is PE38); a protein such as tumor necrosis factor, interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Similarly, the therapeutic agent can be a viral particle, e.g., a recombinant viral particle, that is conjugated (e.g., via a chemical linker) or fused (e.g., via a viral coat protein) to an antibody of the invention.

Active radioisotopes can also be coupled to antibodies or antigen binding fragments thereof of the invention. Radioactive isotopes can be used in diagnostic or therapeutic applications. Radioactive isotopes that can be coupled to the antibodies include, but are not limited to α-, β-, or γ-emitters, or β- and γ-emitters. Such radioactive isotopes include, but are not limited to iodine (¹³¹I or ¹²⁵I), yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (^{99m}Tc), phosphorus (³²P), rhodium (⁸⁸Rh), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), or gallium (⁶⁷Ga). Radioisotopes useful as therapeutic agents include yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), and rhodium (¹⁸⁸Rh). Radioisotopes useful as labels, e.g., for use in diagnostics, include iodine (¹³¹I or ¹²⁵I), indium (¹¹¹In), technetium (^{99m}Tc), phosphorus (³²P), carbon (¹⁴C), and tritium (³H), or one or more of the therapeutic isotopes listed above.

Useful imaging agents with which an antibody or an antibody portion of the invention may be labelled include fluorescent compounds, various enzymes, prosthetic groups, luminescent materials, bioluminescent materials, fluorescent emitting metal atoms, e.g., europium (Eu), and other anthanides, and radioactive materials (described above). Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, b-galactosidase, acetylcholinesterase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a coloured reaction product, which is detectable. An antibody may also be derivatized with a prosthetic group (e.g., streptavidin/biotin and avidin/biotin). For example, an antibody may be derivatized with biotin, and detected through indirect measurement of avidin or streptavidin binding. Examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of bioluminescent materials include luciferase, luciferin, and aequorin.

### Pharmaceutical Compositions:

In another aspect, the present invention provides compositions, e.g., pharmaceutically acceptable compositions, which include an antibody or an antigen binding fragment thereof of the invention formulated together with a pharmaceutically acceptable carrier.

As used herein, "pharmaceutically acceptable carrier" includes any and all solvents, dispersion media, isotonic and absorption delaying agents, and the like that are physiologically compatible. The carrier can be suitable for intravenous, intramuscular, subcutaneous, parenteral, rectal, spinal or epidermal administration (e.g., by injection or infusion).

The compositions of this invention may be in a variety of forms. These include, for example, liquid, semi-solid and solid dosage forms, such as liquid solutions (e.g., injectable and infusible solutions), dispersions or suspensions, liposomes and suppositories. The preferred form depends on the intended mode of administration and therapeutic application. Typical compositions are in the form of injectable or infusible solutions. The mode of administration is parenteral (e.g., intravenous, subcutaneous, intraperitoneal, intramuscular). In some embodiments, the antibody is administered by intravenous infusion or injection. In other embodiments, the antibody is administered by intramuscular or subcutaneous injection.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Therapeutic compositions typically should be sterile and stable under the conditions of manufacture and storage. The composition can be formulated as a solution, microemulsion, dispersion, liposome, or other ordered structure suitable to high antibody concentration. Sterile injectable solutions can be prepared by incorporating the active compound (i.e., antibody or antibody portion) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the provided methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatine.

The antibodies and antigen binding fragments of the present invention can be administered by a variety of methods known in the art, although for many therapeutic applications, the route/mode of administration is intravenous injection or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are generally known to those skilled in the art.

In certain embodiments, an antibody or an antibody portion of the invention may be orally administered, for example, with an inert diluent or an assimilable edible carrier. The compound (and other ingredients if desired) may also be enclosed in a hard or soft shell gelatine capsule, compressed into tablets, buccal tablets, troches, capsules, elixirs, suspensions, syrups, wafers, and the like. To administer an antibody or an antibody fragment of the invention by other than parenteral administration, it may be necessary to coat the compound with, or co-administer the compound with, a material to prevent its inactivation.

The pharmaceutical compositions of the invention can be administered with medical devices known in the art.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

An exemplary, non-limiting range for a therapeutically or prophylactically effective amount of an antibody or an antigen binding fragment of the invention is 0.1-20 mg/kg, or 1-10 mg/kg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

The pharmaceutical compositions of the invention may include a "therapeutically effective" amount of an antibody or an antigen binding fragment of the invention. A "therapeutically effective" amount refers to an amount effective, at dosages and for periods of time necessary, to achieve the desired therapeutic result. A therapeutically effective amount of the modified antibody or antibody fragment may vary according to factors such as the disease state, age, sex, and weight of the individual, and the ability of the antibody or antibody portion to elicit a desired response in the individual. A therapeutically effective amount is also one in which any toxic or detrimental effects of the modified antibody or antibody fragment is outweighed by the therapeutically beneficial effects. A "therapeutically effective dosage" preferably inhibits a measurable parameter, e.g., tumor growth rate by at least about 20%, at least about 40%, at least about 60%, and in some aspects preferably at least about 80% relative to untreated subjects. The ability of a compound to inhibit a measurable parameter, e.g., cancer, can be evaluated in an animal model system predictive of efficacy in human tumors. Alternatively, this property of a composition can be evaluated by examining the ability of the compound to inhibit, such inhibition *in vitro* by assays known to the skilled practitioner.

Also within the scope of the invention are kits comprising an antibody or antigen-binding fragment thereof of the invention. Also included in the invention are kits comprising immunoconjugates comprising an antibody or antigen binding fragment conjugated to an active agent. Further included are kits comprising liposome compositions comprising antibodies or antigen binding fragments thereof of the invention. The kit can include one or more other elements including: instructions for use; other reagents, e.g., a label, a therapeutic agent, or an agent useful for chelating, or otherwise coupling, an antibody to a label or therapeutic agent, or a radioprotective composition; devices or other materials for preparing the antibody for administration; pharmaceutically acceptable carriers; and devices or other materials for administration to a subject. Instructions for use can include instructions for diagnostic applications of the antibodies or antigen-binding fragment thereof to detect huCEACAM1, huCEACAM3 and/or huCEACAM5, *in vitro,* e.g., in a sample, e.g., a biopsy or cells from a subject, or *in vivo.* The instructions can include instructions for therapeutic application including suggested dosages and/or modes of administration in a subject. Other instructions can include instructions on coupling of the antibody to a chelator, a label or a therapeutic agent, or for purification of a conjugated antibody, e.g., from unreacted conjugation components. As discussed above, the kit can include a label or imaging agent, e.g., any of the labels described herein. As discussed above, the kit can include a therapeutic agent, e.g., a therapeutic agent described herein. In some applications the antibody will be reacted with other components, e.g., a chelator or a label or therapeutic agent, e.g., a radioisotope, e.g., yttrium or lutetium. In such cases the kit can include one or more of a reaction vessel to carry out the reaction or a separation device, e.g., a chromatographic column, for use in separating the finished product from starting materials or reaction intermediates.

The kit can further contain at least one additional reagent, such as a diagnostic or therapeutic agent, e.g., a diagnostic or therapeutic agent as described herein, and/or one or more additional antibodies (or fragments thereof), formulated as appropriate, in one or more separate pharmaceutical preparations.

### Uses of the Invention:

The antibodies have *in vitro* and *in vivo* diagnostic, therapeutic and prophylactic utilities. For example, these antibodies can be administered to cells in culture, e.g. *in vitro* or ex vivo, or in a subject, e.g., *in vivo,* to treat, prevent, and/or diagnose a variety of disorders, disease or medical condition. More specifically, antibodies or antigen binding fragments thereof of the invention and compositions comprising the provided antibodies or antigen binding fragments thereof (e.g., immunoconjugates), can be used for:
- infections
- acute and chronic inflammatory diseases
- immunodeficiencies or immunocompromises,
- vaccinations,
- leukocyte expansion and differentiation
- maturation of antigen presenting cells
- transplantation
- cancer/cancer metastasis
- photothermal therapy
- adopted T cell transfer
- wound healing
- leukocyte expansion and differentiation
- diseases associated with T-cell and/or B-cell response
- immune diseases
- auto-immune diseases
- restoration and/or improvement of immune response e.g. after chemotherapeutic treatment
- adoptive immune therapy, e.g. CAR-T cell therapy
- photodynamic therapy (PDT)
- photo-thermal therapy (PTT)

As used herein, the term "subject" is intended to include human and non-human animals. For example, a subject includes a patient (e.g., a human patient, a veterinary patient), having a disorder to be diagnosed, prevented or treated using the antibody or antigen binding fragment thereof of the invention. In one embodiment, the subject is a human subject.

The antibodies or antigen-binding fragments thereof of the invention may be used in combination with other therapies. For example, the combination therapy can include a composition of the present invention co-formulated with, and/or co-administered with, one or more additional therapeutic agents. In other embodiments, the antibodies are administered in combination with other therapeutic treatment modalities, including surgery, radiation, cryosurgery, and/or thermotherapy. Such combination therapies may advantageously utilize lower dosages of the administered therapeutic agents, thus avoiding possible toxicities or complications associated with the various monotherapies.

Labelled antibodies can be used, for example, diagnostically and/or experimentally in a number of contexts, including (i) to isolate a predetermined antigen by standard techniques, such as affinity chromatography or immunoprecipitation; (ii) to detect a predetermined antigen (e.g., in a cellular lysate or cell supernatant) in order to evaluate the abundance and pattern of expression of the protein; (iii) to monitor protein levels in tissue as part of a clinical testing procedure, e.g., to determine the efficacy of a given treatment regimen.

Thus, in another aspect, the present invention provides a diagnostic method for detecting the presence of huCEACAM1, huCEACAM3 and/or huCEACAM5 protein *in vitro* (e.g., in a biological sample, such as a tissue biopsy). The method includes: (i) contacting the sample with a antibody or antigen binding fragment thereof of the invention, or administering to the subject, the antibody or antigen binding fragment thereof of the invention; (optionally (ii) contacting a reference sample, e.g., a control sample (e.g., a control biological sample, such as plasma, tissue, biopsy, or a control subject)); and (iii) detecting formation of a complex between the antibody or antigen binding fragment thereof of the invention and the sample or subject, or the control sample or subject, wherein a change, e.g., a statistically significant change, in the formation of the complex in the sample or subject relative to the control sample or subject is indicative of the presence of huCEACAM1, huCEACAM3 and/or huCEACAM5 in the sample.

Preferably, the antibody or antigen binding fragment thereof of the invention is directly or indirectly labelled with a detectable substance to facilitate detection of the bound or unbound antibody. Suitable detectable substances include various enzymes, prosthetic groups, fluorescent materials, luminescent materials and radioactive materials, as described above and described in more detail below.

Complex formation between the antibody and the antigen can be detected by measuring or visualizing either the antibody (or antibody fragment) bound to the antigen or unbound antibody (or antibody fragment). Conventional detection assays can be used, e.g., an enzyme-linked immunosorbent assays (ELISA), an radioimmunoassay (RIA) or tissue immunohistochemistry. Alternative to labelling the antibody, the presence of the antigen can be assayed in a sample by a competition immunoassay utilizing standards labelled with a detectable substance and an unlabelled antibody. In this assay, the biological sample, the labelled standards and the antigen binding agent are combined and the amount of labelled standard bound to the unlabelled antibody is determined. The amount of antigen in the sample is inversely proportional to the amount of labelled standard bound to the antigen binding agent. Furthermore, the antibody may be used for diagnosis e.g. via fluorescence guided biopsies and as supportive tool in fluorescence guided surgeries.

In the following the invention is explained in more detail by way of examples.

### FIGURES:

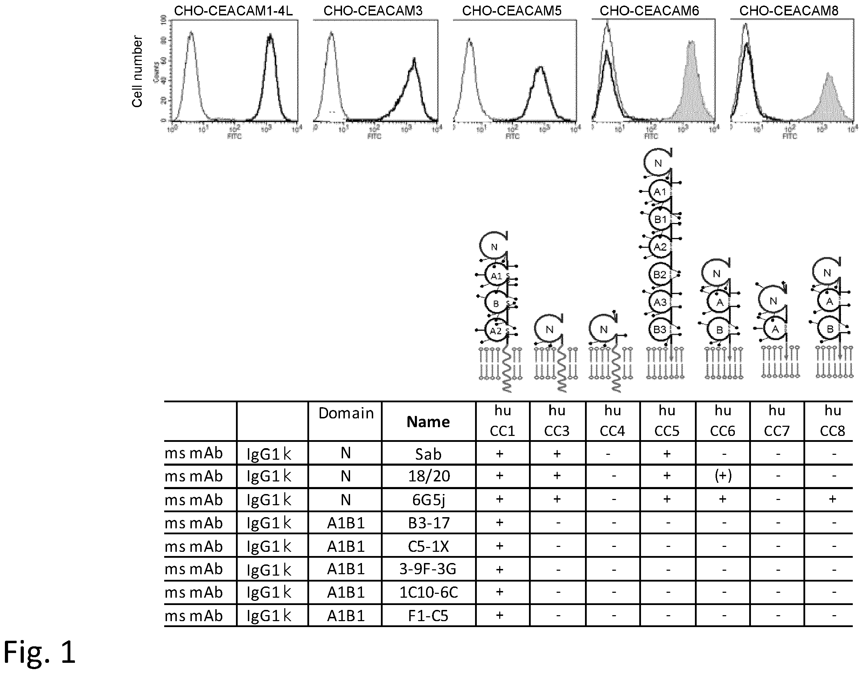

- **Figure 1.**: **Flow cytometric characterization of the CC1/3/5-Sab binding.**
CC1/3/5-Sab was incubated with cell suspensions of CHO transfectants expressing human CEACAM1, CEACAM3, CEACAM5, CEACAM6 and CEACAM8, respectively. After washing, FITC-labeled secondary goat anti mouse antibody was applied. Cell surface binding was analyzed utilizing a FACScalibur flow cytometer (Becton Dickinson). CC1/3/5-Sab binding is shown as thick line, isotype matched IgG as thin line and positive control staining as gray filled histogram. Data show one of three different, representative experiments.

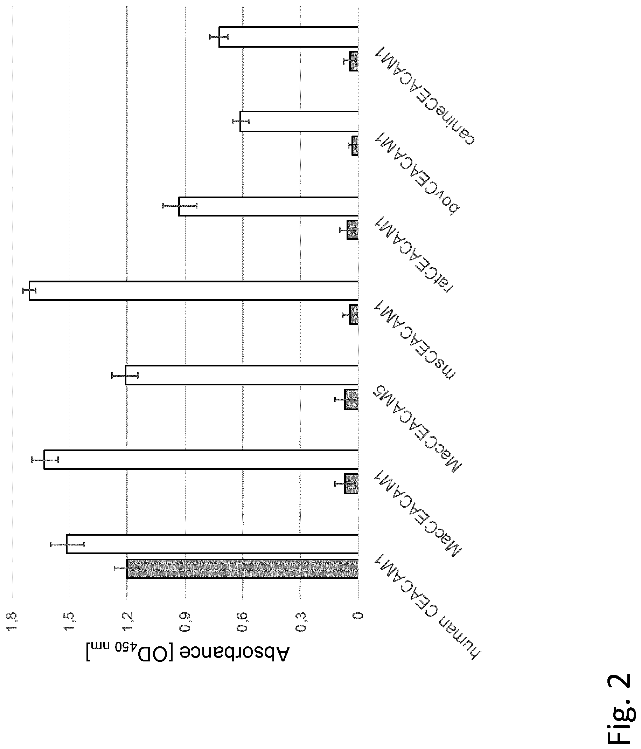

- **Figure 2.**: **ELISA characterization of the CC1/3/5-Sab binding.**
Indicated CHO cell lysates were immobilized on an ELISA plate (NUNC maxisorp). After blocking remaining binding sites with BSA, samples were incubated with CC1/3/5-Sab (grey bars) or positive control staining (white bars) followed by HRP-coupled secondary antibody and TMB substrate development. Enzymatic reaction was stopped by sulfuric acid and measured in a Tecan Sunrise ELISA plate reader at 450 nm. Data are representative of three independent experiments.

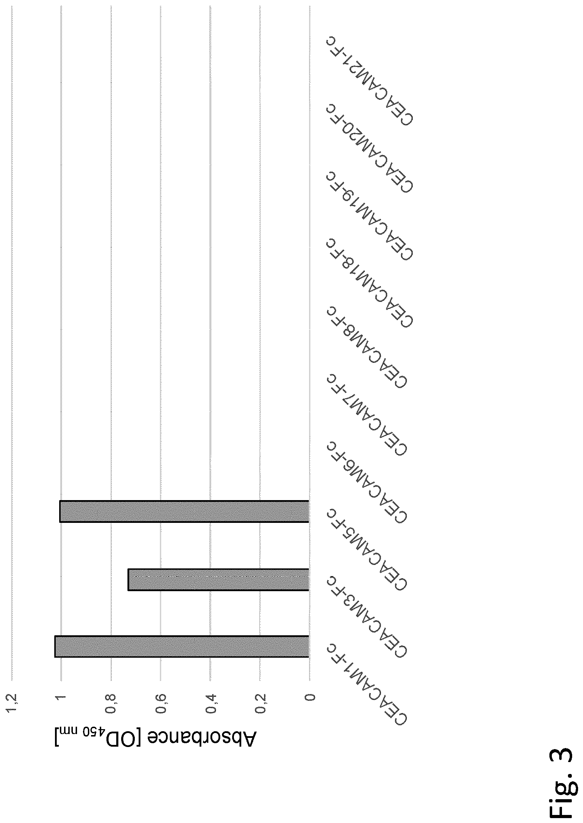

- **Figure 3.**: **Sandwich-ELISA characterization of the CC1/3/5-Sab binding.**
CC1/3/5-Sab were coated on an ELISA plate (NUNC maxisorp). After blocking remaining binding sites with BSA, indicated CEACAM-Fc proteins were incubated. For detection, the HRP-coupled goat anti human Fc antibody was utilized. After washing, TMB substrate was added. The enzymatic reaction was stopped by sulfuric acid and measured in a Tecan Sunrise ELISA plate reader at 450 nm. Data are representative of three independent experiments.

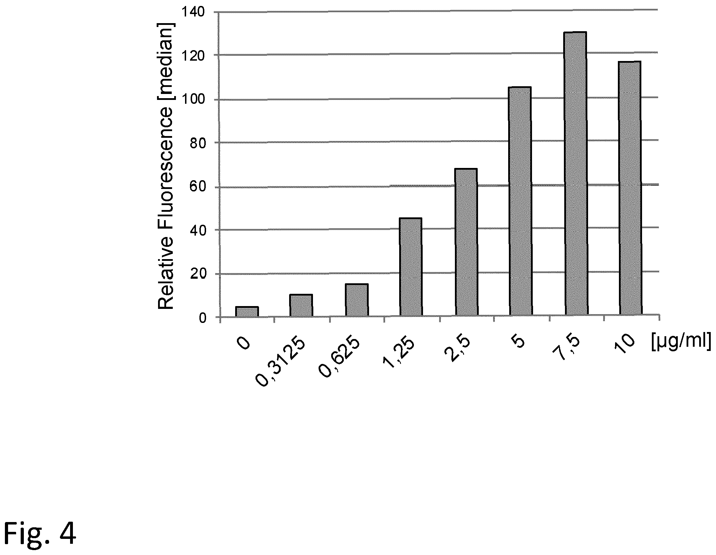

- **Figure 4.**: **Flow cytometric characterization of the CC1/3/5-Sab binding kinetics.**
Increasing concentrations of CC1/3/5-Sab were added to a defined number of CHO-CEACAM1 transfectants. After washing, FITC-labeled secondary goat anti mouse antibody was applied. After washing, samples were analyzed by a FACScalibur flow cytometer (Becton Dickinson). Data show one of three different, representative experiments.

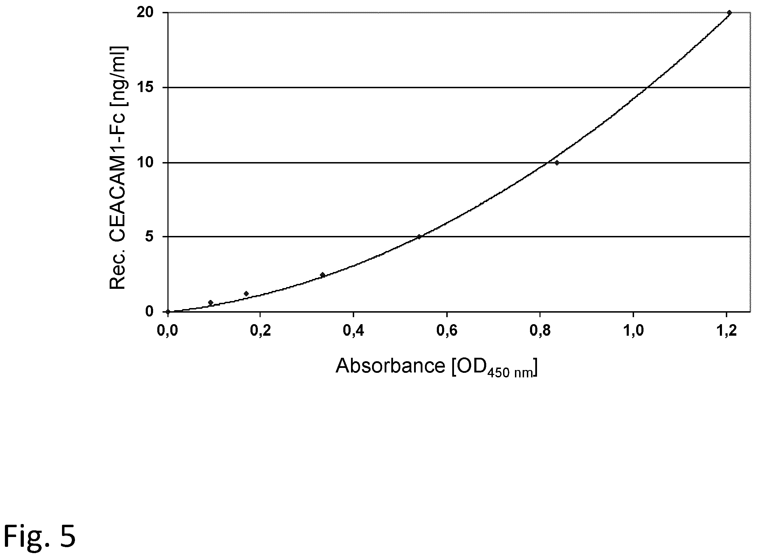

- **Figure 5.**: **Sandwich-ELISA utilizing CC1/3/5-Sab as detector antibody.**
LC-rb pAb CEACAM were coated on an ELISA plate (NUNC maxisorp). After blocking remaining binding sites with BSA, indicated concentrations of CEACAM1-Fc protein were incubated. After washing, CC1/3/5-Sab was added to the samples. Subsequently, HRP-coupled goat anti mouse Ig antibody was used for detection. After washing, TMB substrate was added. The enzymatic reaction was stopped by sulfuric acid and measured in a Tecan Sunrise ELISA plate reader at 450 nm. Data are representative of three independent experiments.
- **Figure 6.**: **Westernblot analyses** of lysates derived from the proliferating (sparse) and confluent human liver cancer cell line HepG2 detected by CC1/3/5-Sab and visualized by HRP-coupled secondary antibody and ECL detection utilizing a Fuiji LAS3000 system.
- **Figure 7.**: **Detection of CEACAMs in human prostate and gastric tissues.**
Paraffin sections of human prostate and gastric tissue were stained with CC1/3/5-Sab (marked by arrow). (A) IHC using indirect immune-peroxidase DAB-technique and (B) indirect fluorescence (IF) labeling was performed. Representative images are shown. Original magnification 200X.
- **Figure 8.**: **Sandwich-ELISA showing that CC1/3/5-Sab binds to the CEACAM-N domain.**
LC-rb pAb CEACAM were coated on an ELISA plate (NUNC maxisorp). After blocking remaining binding sites with BSA, CEACAM1-Fc with the N-domain (grey column) and CEACAM1-dN-Fc (variant lacking the N-domain, white column) was incubated. As negative control served human IgG1 antibody. After washing, CC1/3/5-Sab was added to the samples. Subsequently, HRP-coupled goat anti mouse Ig antibody was used for detection. After washing, TMB substrate was added. The enzymatic reaction was stopped by sulfuric acid and measured in a Tecan Sunrise ELISA plate reader at 450 nm.

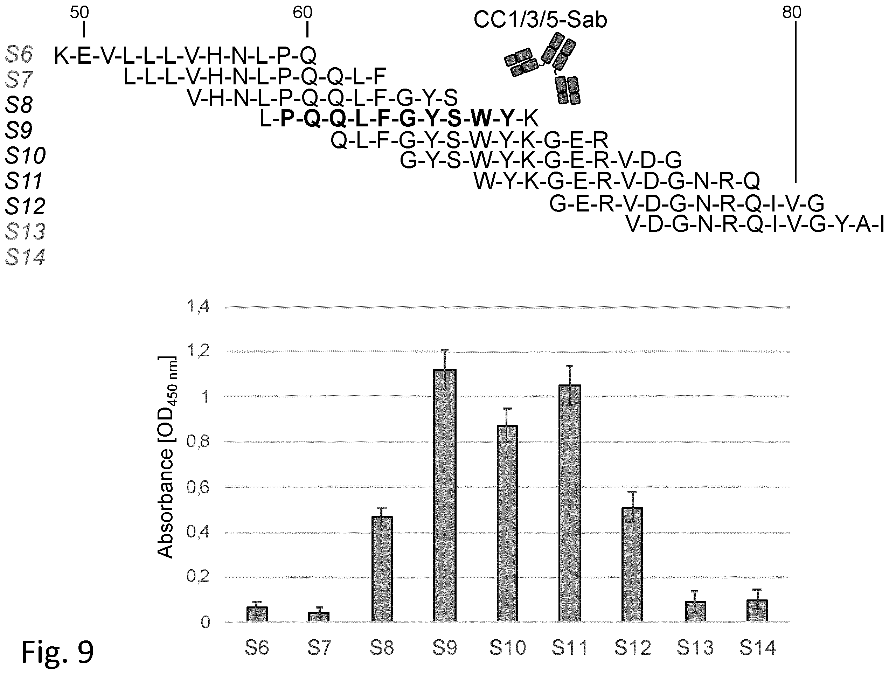

- **Figure 9.**: **ELISA characterization of the CC1/3/5-Sab binding epitope.**
Indicated peptides were spotted to an ELISA plate (NUNC maxisorp). After blocking remaining binding sites with BSA, samples were incubated with CC1/3/5-Sab followed by HRP-coupled secondary antibody and TMB substrate development. Enzymatic reaction was stopped by sulfuric acid and measured in a Tecan Sunrise ELISA plate reader at 450 nm. Data are representative of three independent experiments.

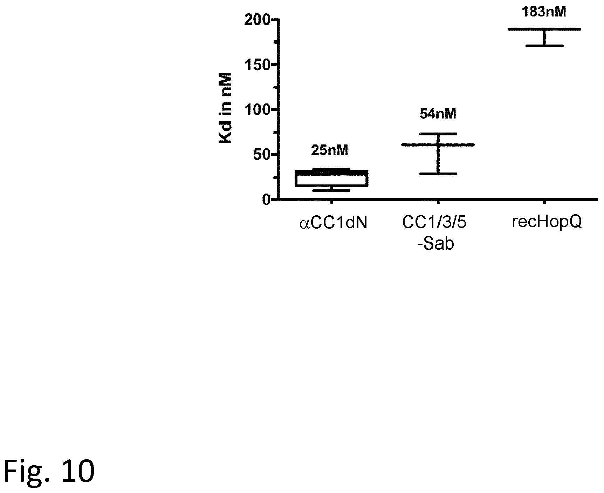

- **Figure 10.**: **Affinity measurement of different CEACAM1 binding proteins.**
Kd measurements of the murine anti CC1dN mAb, CC1/3/5-Sab and recombinant HopQ against immobilized human CEACAM1-Fc fusion protein using the BLItz System from Pall fortéBIO. For each sample, at least three independent Kds were determined and the mean value was calculated.

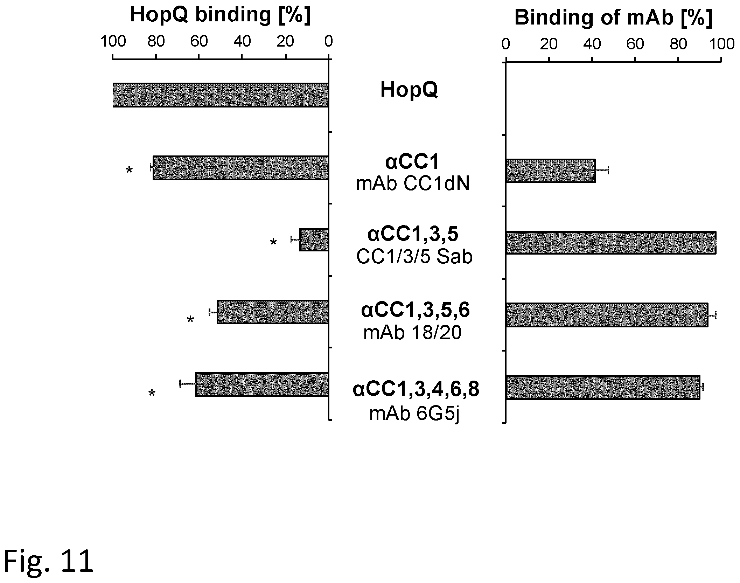

- **Figure 11.**: **Flow cytometric characterization of different anti CEACAM1 antibody competition effects on the binding of HopQ.**
HopQ-PE binding to CHO-CEACAM1 with and without pre-treatment of indicated antibodies was measured by flow cytometry. FITC-coupled goat anti mouse antibody was used to monitor mAb binding. All samples were analyzed by a FACScalibur flow cytometer. Data shown are calculated from three independent experiments while maximum binding of HopQ-PE and mAbs-FITC alone were set 100%, respectively.

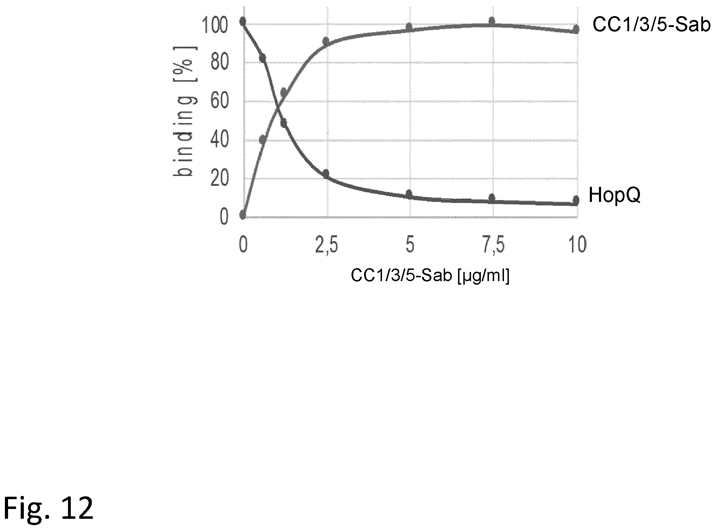

- **Figure 12.**: **Flow cytometric characterization of the CC1/3/5-Sab effect on the binding of HopQ.**
HopQ-PE binding to the human gastric cancer cell line Kato III with and without pre-treatment of indicated antibodies was measured by flow cytometry. FITC-coupled goat anti mouse antibody was used to monitor mAb binding. All samples were analyzed by a FACScalibur flow cytometer (Becton Dickinson). Data shown are calculated from three independent experiments while maximum binding of HopQ-PE and mAbs-FITC alone were set 100%, respectively.

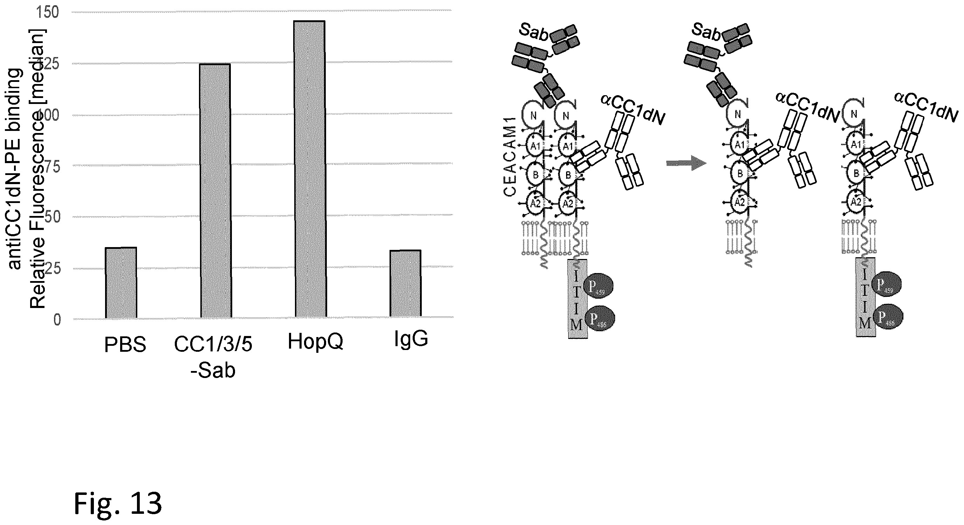

- **Figure 13.**: **Flow cytometric characterization of the CC1/3/5-Sab effect on the binding of CC1dN binding antibodies.**
aCC1dN-PE binding to CHO-huCEACAM1 transfectants with and without pre-treatment of CC1/3/5-Sab or HopQ was measured by flow cytometry. Data shown are representative for at least three independent experiments.

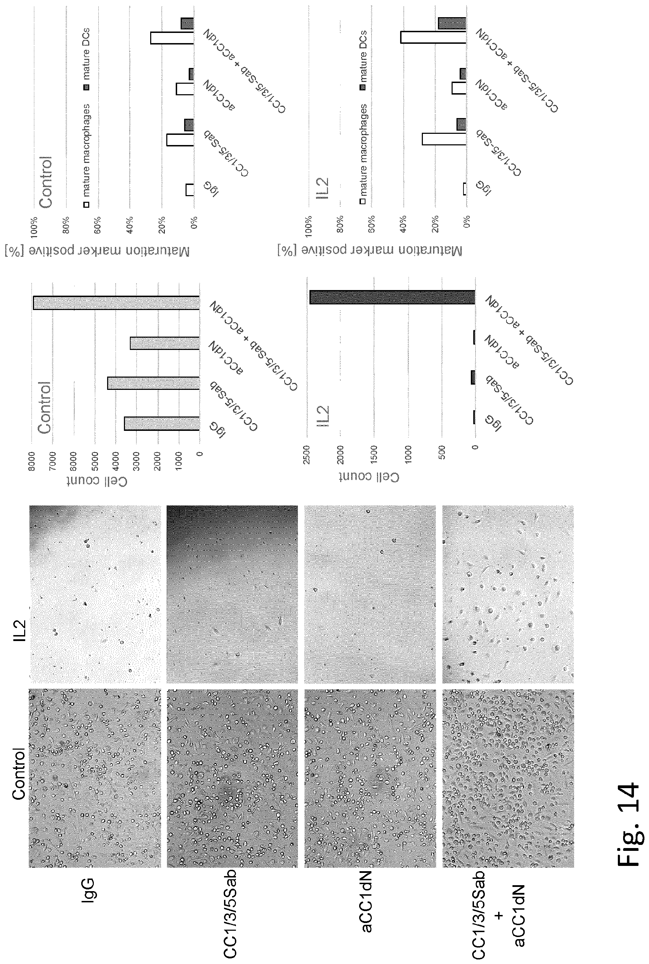

- **Figure 14.**: **Adhesion, differentiation and maturation of PBMC derived monocytes.**
Freshly isolated human PBMCs were cultivated in the absence and presence of IL2 with and without control IgG, CC1/3/5Sab, antiCC1dN mAb and a combination of CC1/3/5Sab + antiCC1dN mAb. After 7 days not attached cells were washed away and microscopic images were taken (left panel). Then adhered cells were collected by trypsinization procedure, counted (mid panel) and analyzed for mature macrophages (25F9⁺ white columns) and mature dendritic cells (CD83⁺, grey columns) by flow cytometry (right panel).

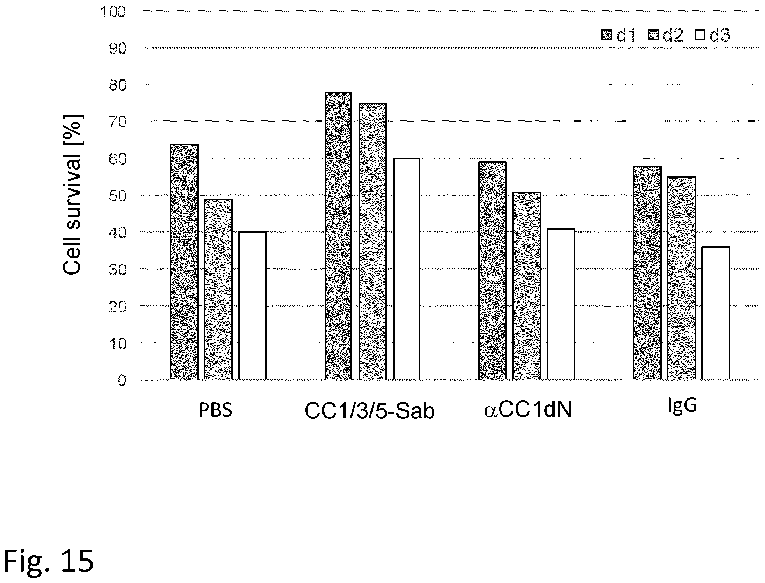

- **Figure 15.**: **Flow cytometric characterization of the cell survival of cold stored cells.**
MKN45 cells were incubated for 1, 2 and 3 days with and without indicated mAbs at 4°C (cold storage). Cells were then harvested and labelled with Annexin V-FITC (Immunotools) and PI (Pierce) to determine the apoptotic rate. Samples were measured by flow cytometry.

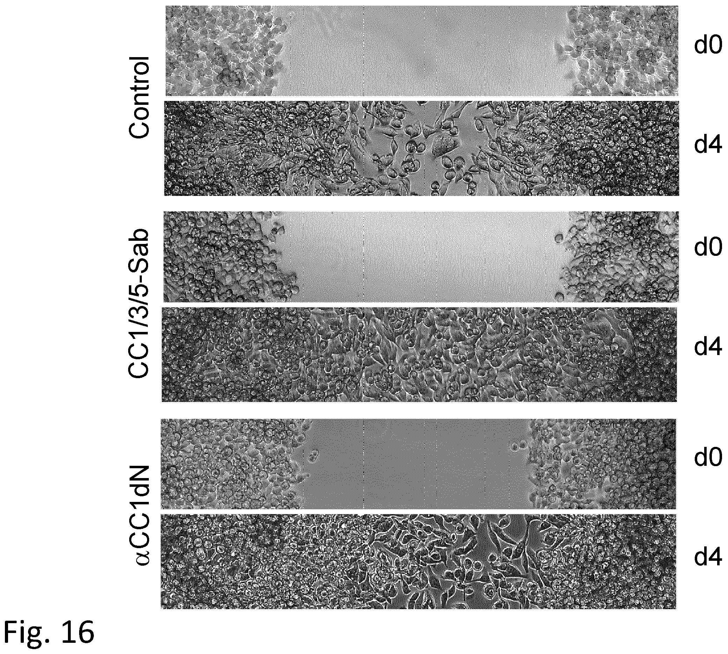

- **Figure 16.**: **Wound healing assay.**
Confluent monolayer of MKN45 cells were scratched and monitored at day 0 (d0). The supernatant was then carefully removed and replaced by media with and without indicated antibodies and incubated for 4 days. Subsequently cells were monitored on day 4 (d4).

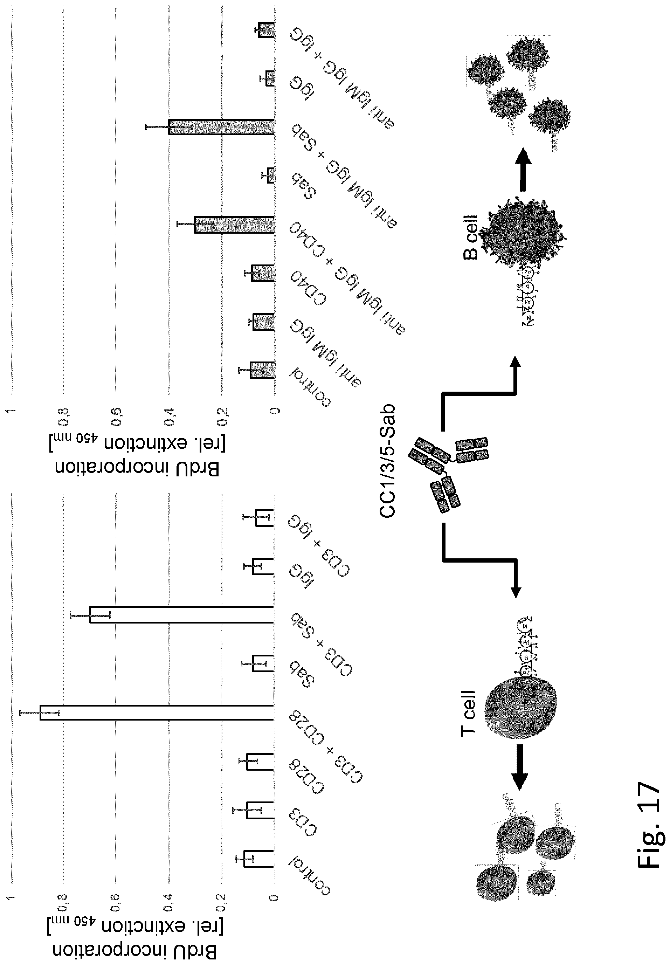

- **Figure 17.**: **Proliferation of differently stimulated PBMCs.**
Freshly isolated PBMCs were incubated with indicated mAbs alone or combinations there of for 3 days. During the last 16 hours the BrdU substrate was added. The assay was developed according to the manufacturer's protocol (Merck Millipore). Proliferation was measured in triplicates by detecting BrdU incorporation via antibody based colorimetric ELISA approach.

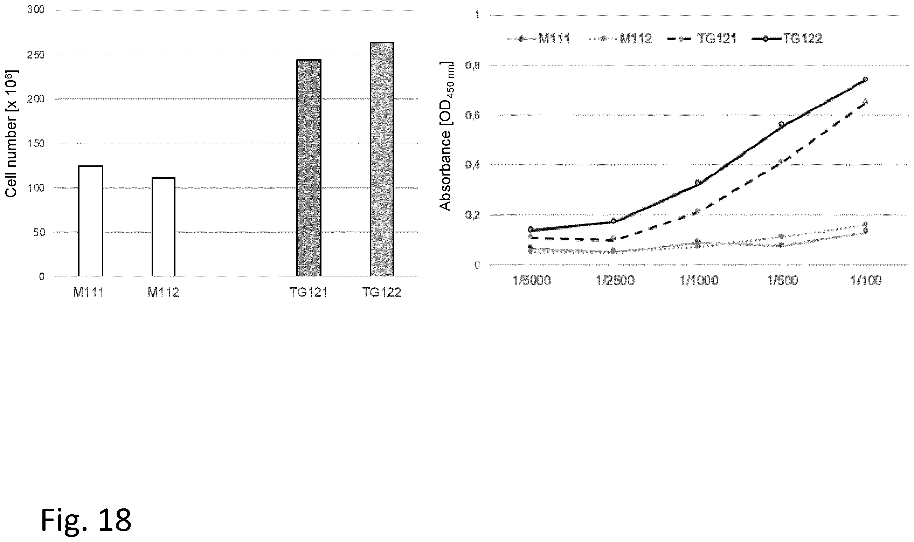

- **Figure 18.**: **Cell number and antigen response of peptide X immunized mice with CC1/3/5-Sab.**
FVB/NJ wt or human CEACAM1-transgenic FVB/NJ mice were immunized three times with peptide X in the presence of CC1/3/5-Sab as specific immune booster. The mice were sacrificed and splenocytes were isolated and counted (left panel).The post-immunization serum was obtained from the blood collected and utilized for ELISA to detect the anti peptide X immune response (right panel). Peptide X was coated to an ELISA plate (NUNC maxisorp). After BSA blocking of remaining binding sites indicated dilutions of the post-immune sera were incubated. Subsequently, HRP-coupled goat anti mouse Ig antibody was used for detection. After washing, TMB substrate was added. The enzymatic reaction was stopped by sulfuric acid and measured in a Tecan Sunrise ELISA plate reader at 450 nm. Data were measured in triplicates.

### EXAMPLES:

### Materials and Methods

### Flow cytometry

Indicated cell types were labeled with CC1/3/5-Sab, 6G5j, aCC1dN, 18/20 (10 µg/ml), mAb 25F9, CD83 and mouse serum, respectively, diluted in 3% FCS/DMEM for 1 h, washed with 3% FCS/DMEM, and incubated with FITC conjugated goat anti-mouse F(ab')2 antibody diluted 1:50 in 3% FCS/DMEM. In some assays directly fluorochrome coupled antibody aCC1dN-PE and recombinant HopQ-PE was used. Background fluorescence was determined using isotype matched Ig. The stained cell samples were examined in a FACScalibur flow cytometer (Becton Dicksinson) and the data were analyzed utilizing the CellQuest software. Where applicable, dead cells, identified by PI staining (5 µg/ml), were excluded from the determination.

For the HopQ binding competition assay CHO-CEACAM1 cells were pre-incubated with indicated CEACAM1 binding antibodies (25 µg/ml or indicated concentrations, 100 µl diluted in 3% FCS/DMEM) for 1 h at RT or left untreated. Then half of the approach was used for the HopQ-PE staining, the other half for the antibody detection utilizing FITC conjugated goat anti-mouse F(ab')2 antibody (Jackson ImmunoResearch) diluted 1:50 in 3% FCS/DMEM. Samples were analyzed by a FACScalibur flow cytometer. Maximum binding measured as relative fluorescence [median value] of HopQ-PE binding alone and mAbs-FITC binding alone were set 100%, respectively. Then inhibition of HopQ-PE binding was calculated. Data shown are calculated from three independent experiments.

### ELISA

Direct ELISA was performed with indicated cell lysates dispensed at 100 µl per well in 96-well microplates (Nunc MaxiSorp, Nalge Nunc). After blocking with 360 µl 1% BSA/PBS, wells were incubated with 100 µl of 5 µg/ml of indicated monoclonal antibody (mAb) or 3 µg/ml rabbit panCEACAM pAb (LeukoCom, Germany) diluted in 0.5% BSA/PBS. Then plates were washed three times with PBS and incubated with HRP-coupled goat anti mouse or anti rabbit antibody (Jackson ImmunoResearch Lab. Inc.). After washing, staining was developed with 100 µl/well tetramethylbenzidine solution (TMB Xtra, EcoTrak) as the color developing reagent. The enzymatic reaction was stopped with 200 mM H₂SO₄ solution after 3-30 minutes and absorbance was measured at 450 nm in a microtiter plate reader (Sunrise Tecan).

### Sandwich-ELISA utilizing CC1/3/5 as (primary) catcher antibody

CC1/3/5-Sab (5 µg/ml diluted in PBS, 100 µl/well) were coated on an ELISA plate (NUNC maxisorp). After blocking remaining binding sites with 1% BSA/PBS, indicated CEACAM-Fc proteins (0.1 µg/ml diluted in 1% BSA/PBS) were incubated for 2 h at RT. For detection, 100 µl HRP-coupled goat anti human Fc antibody diluted 1:10.000 in 1% BSA/PBS was utilized. After washing, TMB substrate was added. The enzymatic reaction was stopped by sulfuric acid and measured in a Tecan Sunrise ELISA plate reader at 450 nm. Assays were performed in triplicates and data shown are representative of three independent experiments.

### Sandwich-ELISA utilizing CC1/3/5 as (secondary) detector antibody

LC-rb pAb CEACAM (5 µg/ml diluted in PBS, 100 µl/well) were coated on an ELISA plate (NUNC maxisorp). After blocking remaining binding sites with 1% BSA/PBS, indicated concentrations of CEACAM1-Fc protein (0-10 µg/ml in 100 µl 1% BSA/PBS) were incubated for 2 h at RT. After washing, CC1/3/5-Sab (5 µg/ml diluted in 1% BSA/PBS, 100 µl/well) was added to the samples for 1 h at RT. Subsequently, 100 µl HRP-coupled goat anti mouse Ig antibody diluted 1:20.000 in 1% BSA/PBS was used for detection. After washing, TMB substrate was added. The enzymatic reaction was stopped after 5-20 min by sulfuric acid and measured in a Tecan Sunrise ELISA plate reader at 450 nm. Assays were performed in triplicates and data shown are representative of three independent experiments.

### ELISA characterization of the CC1/3/5-Sab binding epitope

Overlapping peptides with indicated sequence derived from the human CEACAM1-N domain were spotted to an ELISA plate. After blocking remaining binding sites with 1% BSA, samples were incubated with CC1/3/5-Sab (5 µg/ml diluted in 1% BSA/PBS, 100 µl/well) followed by 100 µl HRP-coupled goat anti mouse Fc antibody diluted 1:10.000 in 1% BSA/PBS and TMB substrate development (100 µl/well). Enzymatic reaction was stopped by sulfuric acid and measured in a Tecan Sunrise ELISA plate reader at 450 nm. Assays were performed in triplicates and data shown are representative of three independent experiments.

### Westernblot

Endogenous CEACAM 1 expressing cells grown in log phase (proliferating) and tight confluent, contact inhibited stage were lysed in RIPA based lysis buffer containing 50 mM Tris-HCI, pH 7.5, 150 mM NaCl, 1% Triton X-100, 0.5% sodium deoxycholate supplemented with protease inhibitor cocktail set III (Calbiochem) and PhosSTOP phosphatase inhibitor cocktail (Roche) on ice for 30 min. Lysates were centrifuged at 10,000 g and 4°C for 15 min and approximately 50 µg of total protein was subjected to SDS-PAGE, blotted to nitrocellulose membrane (Schleicher & Schuell, Dassel, Germany) and reacted with CC1/3/5-Sab. After washing HRP-coupled goat anti mouse pAb was added and subsequently detected by ECL chemiluminescence substrate and monitored by the LAS3000 gel documentation system (Fuji).

### Immunohistochemistry (IHC) and Immunofluorescence (IF)

Serial sections of 4 µm were prepared from 4% paraffin-embedded indicated human prostate and gastric tissues previously fixed in neutrally buffered formalin and mounted on 3-aminopropyltriethoxysilane-coated slides were demasked (10 min at 95°C in citrate buffer pH 6). Endogenous peroxidase activity was blocked by treatment with 3% H₂O₂ for 5 minutes and subsequent washing with PBS. Then sections were blocked with 1% BSA/PBS and stained with 10 µg/ml CC1/3/5Sab followed either by HRP- (IHC, left panel) or DL488- (IF, right panel) coupled goat anti mouse IgG Fab2 antibody (Jackson ImmunoResearch Lab. Inc.). The samples were washed after each staining step with cold PBS three times. HRP-staining was developed with 3,3-diaminobenzidine (DAB, brown precipitate) and the reaction was stopped by several washes with PBS. Stained sections were mounted and documented by microscopy. Fluorescent staining was analyzed with the Leica DMI4000B microscope.

### Affinity determination

The Kd of the murine mAb and recombinant HopQ against human CEACAM1-huFc fusion protein was determined in real time by interferometry Bio-layer detection using the BLItz System from Pall fortéBIO (Pall Life Sciences, USA). Recombinant CEACAM1-huFc was immobilized on an Anti-Human IgG Fc capture sensor (AHC) sensor (#18-5060) (120 sec) and excess protein was removed by washing with PBS (80 sec). For the Kon determination, samples were added at a concentration ranging from 7 nM to 3333 nM (120 sec) and the Koff was determined by washing with PBS until a stable binding curve was achieved (120 sec). The Kd was calculated by the average of the kd-values taken at different concentrations (global fitting) and using a PBS control-run as reference. For each sample, the final Kd was determined in three independent experiments and the average calculated.

### Phase contrast imaging of wound healing assay and cell adhesion

Confluent MKN45 cell cultures were grown in a 24 well cell culture plate for 48 hours. Wounds were made with the tip of a micropipette. Cells were maintained in cell culture media in the presence or absence of 5 µg/ml CC1/3/5-Sab and antiCC1dN mAb, respectively. To analyze cell motility, phase contrast microscopy was done.

Phase contrast microscopy of adherent human PBMC generated macrophages and dendritic cells (DCs) was performed by treating freshly isolated PBMCs with IgG, CC1/3/5-Sab3, anti CC1dN and the combination of CC1/3/5-Sab3 + anti CC1dN in the presence or absence of interleukin 2 (IL2, Immunotools, 300 IU/ml) for 7 days at 37°C in a humidified atmosphere with 5% CO₂. Adherence and cellular morphology after the different stimulations were monitored by standard phase contrast microscopy utilizing the Leica DMIL-system (Leica Microsystems, Wetzlar, Germany) and the ProgRes Capture Pro2.5 analyses software (Jenoptik, Jena, Germany).

### Manual cell counting with the Neubauer chamber

For determination of the absolute cell number a Neubauer haemocytometer chamber was used. Depending on the type of sample, a preparation of a dilution with a suitable concentration was prepared for cell counting (e.g. a dilution of 1:100). A volume of 10 µl was filled via capillary action in a counting chamber. Using the 10 x objective the total number of cells found in 4 large corner squares was counted and used for further calculations.

### Isolation of Peripheral Blood Mononuclear Cells (PBMCs)

Peripheral blood was collected into heparinized tubes and mononuclear cells were isolated using Ficoll-Hypaque (ρ = 1.077 g/mL) density gradient centrifugation at 400 xg for 30 min. The buffy coat containing mononuclear cells was isolated, transferred to a fresh centrifuge tube, and washed twice with PBS (using ≈3 vol of collected buffy coat each time). The final cell pellet containing peripheral blood mononuclear cells (PBMC) was resuspended to a final level of 1-2 x 10⁶ cells/mL in RPMI 1640 medium supplemented with L-glutamine, 10% heat-inactivated fetal bovine serum, sodium pyruvate, 100 U penicillin/mL, and 0.1 mg streptomycin/mL (all Gibco, Paisley, UK).

### BrdU cell proliferation study of differently stimulated human PBMCs

Cell proliferation assay was performed by using the BrdU Cell Proliferation Assay kit (Calbiochem) according to the manufacturer's instruction. Freshly isolated PBMC were plated at a density of 25,000 cells/well in triplicate in 96-well tissue culture microtiter plates (Nunc, Denmark) and incubated for 48 hours at 37°C and 5% CO₂ with and without CD3 (Okt3, 10 ng/ml) CD28 (10 µg/ml), CC1/3/5-Sab (10 µg/ml), IgG (10 µg/ml) anti IgG/IgM (10 µg/ml), CD40 (10 µg/ml) or combinations thereof as indicated. After the corresponding period, BrdU label was added into all wells and incubated for an additional 24 hours. Thereafter, the cells were centrifuged at 300 xg for 10 min, the labeling solution was removed, and the plate then was dried at 60°C for 1 hr before the cells were fixed. Then, fixative/denaturing solution was loaded to fix the cells on the plate for 30 minutes followed by addition of anti-BrdU antibody into the wells. The wells were washed thrice with PBS to remove the non-specific binding and then the conjugate was added into the wells. After 30 minutes, the 3,3',5,5'-Tetramethylbenzidine (TMB) substrate solution was loaded in dark condition at RT. After adding the 0.16 M sulfuric acid stop solution, the plate was read by Sunrise ELISA Reader (Tecan) at the wavelength of 450 nm.

### Results:

Here we present data characterizing a novel human CEACAM binding mouse monoclonal IgG kappa antibody (mAb) named CC1/3/5-Sab. The binding fragment of CC1/3/5-Sab has been determined to be characterized by three heavy chain complementarity determining regions (HCDR1, HCDR2, and HCDR3), and three light chain complementarity determining regions (LCDR1, LCDR2, and LCDR3), wherein
HCDR1 comprises the amino acid sequence of SEQ ID NO:1;
HCDR2 comprises the amino acid sequence of SEQ ID NO:2; and
HCDR3 comprises the amino acid sequence of SEQ ID NO:3; and wherein

LCDR1 comprises the amino acid sequence of SEQ ID NO:4;
LCDR2 comprises the amino acid sequence of GAT or GATX as in SEQ ID NO:5; and
LCDR3 comprises the amino acid sequence of SEQ ID NO:6.

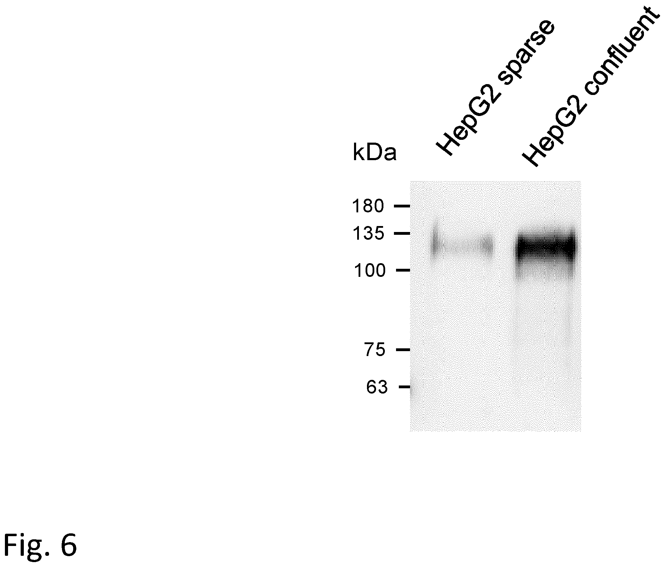

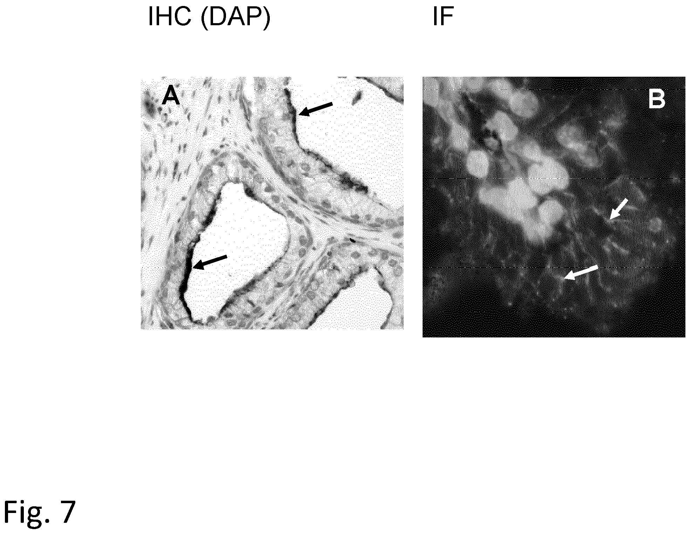

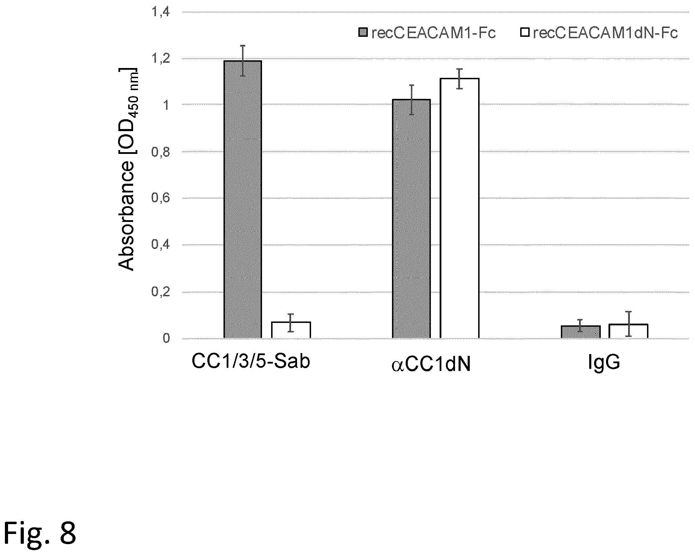

It has been found that CC1/3/5-Sab specifically recognizes human CEACAM1, CEACAM3 and CEACAM5 (also known as CEA, Figure 1). It does not cross-react with CEACAMs in other species like mouse, rat and macaque (Figure 2). Beside CEACAM1/3/5 it does not bind other CEACAMs namely CEACAM4-21 (Figure 3). The dose-response curve (Figure 4), kinetics (data not shown) and Kd determination (Figure 10) revealed a high affine binding of CC1/3/5-Sab. Consequently CC1/3/5-Sab can be utilized in flow cytometry, ELISA, westernblot, immune precipitation, immune histology, fluorescence microscopy and a wide variety of functional assays *in vitro* and *in vivo* (Figure 1-18). Further analysis using full length CEACAM1-Fc proteins and CEACAM1-dN-Fc lacking the N domain revealed the binding of CC1/3/5-Sab to the N domain of human CEACAM1 (Figure 8). Overlapping peptide analyzes utilizing peptide fragments derived from the human CEACAM1 N domain sequence revealed the epitope of CC1/3/5-Sab antibody to comprise the amino acid sequence P-Q-Q-L-F-G-Y-S-W-Y (SEQ ID NO: 11). In addition, we determined the CDRs in the variable like domains of CC1/3/5-Sab and cloned human IgG1, IgG2a and IgG4 variants of CC1/3/5-Sab having same properties as the murine CC1/3/5-Sab.

Further analyzes of the binding site of CC1/3/5-Sab revealed that it is the sole one so far identified blocking entirely the binding of HopQ in a dose dependent manner (Figure 11, Figure 12). HopQ is a bacterial protein expressed by *Helicobacter pylori* used to interact with the N domain of various CEACAMs expressed in human epithelia, endothelia and leukocyte subtypes. Important to note, HopQ analogues exist binding to exact the same binding side in the N domain of human CEACAM1 were also identified named UspA1 of *Moraxella catarrhalis, Neisserial* Opa proteins, the trimeric autotransporter adhesin CbpF of *Fusobacterium* spp. and other pathogens. Thus, CC1/3/5-Sab is able to inhibit the specific interaction of pathogenic ligands and consequently prevent infectious actions.

Next we found that binding of CC1/3/5-Sab to membrane anchored CEACAM1 leads to dissolution of the cis-dimeric/oligomeric organization of CEACAM1 as monitored by the binding of an antibody (anti CC1dN, anti human CEACAM1 antibody binding the A1/B domains) having its epitope in a CEACAM1 region not accessible as long as the CEACAMs are in cis-dimeric/oligomeric organization (Figure 13). It seems to recognize the monomeric appearance of CEACAM1 on the cell surface. As such, CEACAM1 is able to interact with one of its co-receptors e.g. the B and T cell receptor, TLR2- and 4, EGFR, insulin receptor, G-CSFR, VEGFR1-3 and others. If these have bound to their specific ligands, monomeric CEACAM1 seems to modulate their functions. Interestingly, generation of monomeric CEACAM1 by CC1/3/5-Sab in combination with the anti CC1dN antibody leads to differentiation and maturation of macrophages and dendritic cells as shown by increased adherence to the cell culture plate as well as the presence of maturation markers for macrophages and dendritic cells (Figure 14). Thus, CC1/3/5-Sab at least in combination with antiCC1dN triggers the maturation of antigen presenting cells crucial for the resolution of infections, to combat tumor cells and indispensable for vaccinations and other immunological processes. Beside, application of CC1/3/5-Sab to epithelial cells and leukocytes leads to increased survival rate if stored e.g. at 4°C for storage (Figure 15) or upon stimulation. CC1/3/5-Sab also supports wound healing processes (Figure 16) and appears as co-stimulatory/modulatory factor for T and B cell proliferation (Figure 17). This pro-inflammatory effect was not only seen in *in vitro* experiments but also in *in vivo* assays utilizing a human CEACAM1 transgenic mouse system for peptide immunization. As expected, peptides alone were poor immunogenic whereas in the presence of CC1/3/5-Sab a sufficient immune reaction could be detected with respect to the amount of splenocytes and peptide specific antibodies in the post-immune sera of the mice (Figure 18). Thus, CC1/3/5-Sab regulates immune reactions on the T- and B-lymphocyte level as well as antigen presenting cell level (e.g. macrophages and DCs) and therefore can support therapeutic approaches on the level of vaccination e.g. as novel detergent, tumor immune treatment (potentially to support PD1/PDL1 and other immune checkpoint approaches), treatment of infections, wound healing, for *in vitro* and *in vivo* immune cell expansion for T-CAR, tumor specific DCs therapy in which DCs are collected near the primary tumor and then *in vitro* expanded in the presence of tumor antigens and later infused into the patient for post-operative tumor treatment, *in vitro* expansion of adaptive leukocytes to preserve the immunological memory to be given back to the patient after chemo-/radiotherapy, for expanding e.g. virus specific B cells for subsequent immortalization for the production of human B lymphocytes that are capable of secreting corresponding antibodies in unlimited quantities.

Taken together, binding the N domain of human CEACAM1, CEACAM3 and CEACAM5 exact in a binding site crucial for bacterial adhesins, CC1/3/5-Sab is a unique monoclonal antibody supporting anti-apoptotic, wound healing and immune-regulatory (T cell, B cell and ag-presenting cell level) processes.

## Claims

1. An isolated antibody or antigen binding fragment thereof binding specifically to an epitope on huCEACAM1/3/5 comprising or consisting of the amino acid sequence of SEQ ID NO:11.

2. The isolated antibody or antigen binding fragment thereof of claim 1, wherein the isolated antibody or antigen binding fragment comprises three heavy chain complementarity determining regions (HCDR1, HCDR2, and HCDR3), and three light chain complementarity determining regions (LCDR1, LCDR2, and LCDR3), wherein
HCDR1 comprises the amino acid sequence of SEQ ID NO:1;
HCDR2 comprises the amino acid sequence of SEQ ID NO:2; and
HCDR3 comprises the amino acid sequence of SEQ ID NO:3; and wherein
LCDR1 comprises the amino acid sequence of SEQ ID NO:4;
LCDR2 comprises the amino acid sequence of GAT or GATX as in SEQ ID NO:5; and
LCDR3 comprises the amino acid sequence of SEQ ID NO:6.

3. The isolated antibody or antigen binding fragment thereof of any one of the preceding claims, wherein the antibody or antigen binding fragment comprises a heavy chain comprising the amino acid sequence of SEQ ID NO:7, and a light chain comprising the amino acid sequence of SEQ ID NO:8.

4. The isolated antibody or antigen binding fragment thereof of any one of the preceding claims, wherein the antibody is monoclonal.

5. The isolated antibody or antigen binding fragment thereof of any one of the preceding claims,
wherein the antibody is recombinant; or
wherein the antibody is an IgG, IgM, IgA or an antigen binding fragment thereof; or
wherein the antibody is a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, or a monovalent.

6. The isolated antibody or antigen binding fragment thereof of any one of the preceding claims, wherein the antibody is a humanized antibody or de-immunized antibody.

7. The isolated antibody or antigen binding fragment thereof of any one of the preceding claims, wherein the antibody is conjugated to an active agent, preferably to an imaging agent, a therapeutic agent, a toxin or a radionuclide.

8. A pharmaceutical composition comprising the isolated antibody or antigen binding fragment thereof of any one of claims 1 to 7 and a pharmaceutically or physiologically acceptable carrier or excipient.

9. A polynucleotide molecule comprising a nucleic acid sequence encoding an isolated antibody or antigen binding fragment thereof of any one of claims 1 to 7.

10. A host cell comprising one or more polynucleotide molecule(s) encoding an isolated antibody or antigen binding fragment thereof of any one of claims 1 to 7, optionally wherein the host cell is a mammalian cell, a yeast cell, a bacterial cell, a ciliate cell or an insect cell.

11. A method of manufacturing an antibody comprising:
(a) expressing one or more polynucleotide molecule(s) encoding an isolated antibody or antigen binding fragment thereof of any one of claims 1 to 7 in a cell; and
(b) purifying the antibody from the cell.

12. A kit comprising an isolated antibody or antigen binding fragment of any of claims 1 to 7, and instructions for use of the antibody, optionally further wherein the antibody is lyophilized.

13. An isolated antibody or antigen binding fragment of any of claims 1 to 7, or a pharmaceutical composition of claim 8, for use in a diagnostic method of a disease or medical condition.

14. An isolated antibody or antigen binding fragment of any of claims 1 to 7, or a pharmaceutical composition of claim 8, for use in treating or preventing a disease or medical condition.

15. An antibody or antigen binding fragment for use according to claim 13 or 14, or a pharmaceutical composition for use according to claim 13 or 14, wherein the disease or medical condition is selected from infection, acute and chronic inflammatory diseases, immunodeficiencies or immunocompromises, vaccinations, leukocyte expansion and differentiation, maturation of antigen presenting cells, transplantation, cancer/cancer metastasis, photothermal therapy, adopted T cell transfer, wound healing, leukocyte expansion and differentiation, diseases associated with T-cell and/or B-cell response, immune diseases, auto-immune diseases, restoration and/or improvement of immune response, adoptive immune therapy, e.g. CAR-T cell therapy, photodynamic therapy (PDT), photo-thermal therapy (PTT).


SEQUENCE LISTING

<110> LeukoCom GmbH

<120> A novel antibody binding specifically to human CEACAM1/3/5 and use
thereof

<130> P25629EP

<160> 12

<170> BiSSAP 1.3.6

<210> 1
<211> 8
<212> PRT
<213> Artificial Sequence

<220>
<223> HCDR1

<400> 1
Gly Tyr Thr Phe Thr Thr Tyr Val
1               5

<210> 2
<211> 8
<212> PRT
<213> Artificial Sequence

<220>
<223> HCDR2

<400> 2
Phe Asn Pro Tyr Asn Asp Gly Thr
1               5

<210> 3
<211> 11
<212> PRT
<213> Artificial Sequence

<220>
<223> HCRD3

<400> 3
Ala Arg Trp Ala Tyr Asp Gly Ser Tyr Ala Tyr
1               5                   10

<210> 4
<211> 6
<212> PRT
<213> Artificial Sequence

<220>
<223> LCDR1

<400> 4
Asp His Ile Asn Asn Trp

1               5

<210> 5
<211> 4
<212> PRT
<213> Artificial Sequence

<220>
<223> LCDR2, wherein X at position 4 can be any amino acid

<400> 5
Gly Ala Thr Xaa
1

<210> 6
<211> 9
<212> PRT
<213> Artificial Sequence

<220>
<223> LCDR3

<400> 6
Gln Gln Tyr Trp Arg Thr Pro Phe Thr
1               5

<210> 7
<211> 118
<212> PRT
<213> Artificial Sequence

<220>
<223> VH with HCDR1 to 3

<400> 7
Glu Val Gln Leu Gln Gln Ser Gly Pro Glu Leu Val Lys Pro Gly Thr
1               5                   10                  15
Ser Val Lys Met Ser Cys Lys Ala Ser Gly Tyr Thr Phe Thr Thr Tyr
            20                  25                  30
Val Met His Trp Val Gln Gln Lys Pro Gly Gln Gly Leu Asp Trp Ile
        35                  40                  45
Gly Phe Phe Asn Pro Tyr Asn Asp Gly Thr Lys Tyr Asn Glu Lys Phe
    50                  55                  60
Lys Gly Lys Ala Thr Leu Thr Ser Asp Lys Ser Ser Ser Thr Ala Tyr
65                  70                  75                  80
Met Glu Leu Ser Ser Leu Thr Ser Glu Asp Ser Ala Val Tyr Tyr Cys
                85                  90                  95
Ala Arg Trp Ala Tyr Asp Gly Ser Tyr Ala Tyr Trp Gly Gln Gly Thr
            100                 105                 110
Thr Leu Thr Val Ser Ser
        115

<210> 8
<211> 107
<212> PRT
<213> Artificial Sequence

<220>
<223> VL with LCDR1 to 3

<400> 8
Asp Ile Gln Met Thr Gln Ser Ser Ser Tyr Leu Ser Val Phe Leu Gly
1               5                   10                  15
Gly Arg Val Thr Ile Thr Cys Lys Ala Ser Asp His Ile Asn Asn Trp
            20                  25                  30
Leu Ala Trp Tyr Gln Gln Lys Pro Gly Asn Ala Pro Arg Leu Leu Ile
        35                  40                  45
Ser Gly Ala Thr Ser Leu Glu Thr Gly Val Pro Ser Arg Phe Ser Gly
    50                  55                  60
Ser Gly Ser Gly Lys Asp Tyr Thr Leu Ser Ile Ile Ser Leu Gln Thr
65                  70                  75                  80
Glu Asp Val Ala Thr Tyr Tyr Cys Gln Gln Tyr Trp Arg Thr Pro Phe
                85                  90                  95
Thr Phe Gly Ser Gly Thr Lys Leu Glu Ile Lys
            100                 105

<210> 9
<211> 354
<212> DNA
<213> Artificial Sequence

<220>
<223> encoding VH

<400> 9
gaggtccagc tgcagcaatc tggacctgag ctggtaaagc ctgggacttc agtgaagatg      60

tcctgcaagg cttctggata cacattcact acctatgtta tgcactgggt gcaacagaag     120

cctgggcagg gccttgactg gattggattt tttaatcctt acaatgatgg tactaagtac     180

aatgagaagt tcaaaggcaa ggccacactg acttcagaca aatcctccag cacagcctac     240

atggaactca gcagcctgac ctctgaggac tctgcggtct attactgtgc aagatgggcc     300

tacgatggta gctacgccta ctggggccaa ggcaccactc tcacagtctc ctca           354

<210> 10
<211> 321
<212> DNA
<213> Artificial Sequence

<220>
<223> encoding VL

<400> 10
gatatccaga tgacacaatc ttcatcctac ttgtctgtat ttctaggagg cagagtcacc      60

attacttgca aggcaagtga ccacattaat aattggttag cctggtatca gcagaaacca     120

ggaaatgctc ctaggctctt aatatctggg gcaaccagtt tggaaactgg ggttccttca     180

agattcagtg gcagtggatc tggaaaggat tacactctca gcattatcag tcttcagact     240

gaagatgttg ctacttatta ctgtcaacag tattggagaa ctccattcac gttcggctcg     300

gggacaaagt tggaaataaa a                                               321

<210> 11
<211> 10
<212> PRT
<213> Homo sapiens

<220>
<223> fragment of huCEACAM1, epitope of antibody of the invention

<400> 11
Pro Gln Gln Leu Phe Gly Tyr Ser Trp Tyr
1               5                   10

<210> 12
<211> 526
<212> PRT
<213> Homo sapiens

<220>
<223> CEACAM1

<400> 12
Met Gly His Leu Ser Ala Pro Leu His Arg Val Arg Val Pro Trp Gln
1               5                   10                  15
Gly Leu Leu Leu Thr Ala Ser Leu Leu Thr Phe Trp Asn Pro Pro Thr
            20                  25                  30
Thr Ala Gln Leu Thr Thr Glu Ser Met Pro Phe Asn Val Ala Glu Gly
        35                  40                  45
Lys Glu Val Leu Leu Leu Val His Asn Leu Pro Gln Gln Leu Phe Gly
    50                  55                  60
Tyr Ser Trp Tyr Lys Gly Glu Arg Val Asp Gly Asn Arg Gln Ile Val
65                  70                  75                  80
Gly Tyr Ala Ile Gly Thr Gln Gln Ala Thr Pro Gly Pro Ala Asn Ser
                85                  90                  95
Gly Arg Glu Thr Ile Tyr Pro Asn Ala Ser Leu Leu Ile Gln Asn Val
            100                 105                 110
Thr Gln Asn Asp Thr Gly Phe Tyr Thr Leu Gln Val Ile Lys Ser Asp
        115                 120                 125
Leu Val Asn Glu Glu Ala Thr Gly Gln Phe His Val Tyr Pro Glu Leu
    130                 135                 140
Pro Lys Pro Ser Ile Ser Ser Asn Asn Ser Asn Pro Val Glu Asp Lys
145                 150                 155                 160
Asp Ala Val Ala Phe Thr Cys Glu Pro Glu Thr Gln Asp Thr Thr Tyr
                165                 170                 175
Leu Trp Trp Ile Asn Asn Gln Ser Leu Pro Val Ser Pro Arg Leu Gln
            180                 185                 190
Leu Ser Asn Gly Asn Arg Thr Leu Thr Leu Leu Ser Val Thr Arg Asn
        195                 200                 205
Asp Thr Gly Pro Tyr Glu Cys Glu Ile Gln Asn Pro Val Ser Ala Asn
    210                 215                 220
Arg Ser Asp Pro Val Thr Leu Asn Val Thr Tyr Gly Pro Asp Thr Pro
225                 230                 235                 240
Thr Ile Ser Pro Ser Asp Thr Tyr Tyr Arg Pro Gly Ala Asn Leu Ser
                245                 250                 255
Leu Ser Cys Tyr Ala Ala Ser Asn Pro Pro Ala Gln Tyr Ser Trp Leu
            260                 265                 270
Ile Asn Gly Thr Phe Gln Gln Ser Thr Gln Glu Leu Phe Ile Pro Asn
        275                 280                 285
Ile Thr Val Asn Asn Ser Gly Ser Tyr Thr Cys His Ala Asn Asn Ser
    290                 295                 300
Val Thr Gly Cys Asn Arg Thr Thr Val Lys Thr Ile Ile Val Thr Glu
305                 310                 315                 320

Leu Ser Pro Val Val Ala Lys Pro Gln Ile Lys Ala Ser Lys Thr Thr
                325                 330                 335
Val Thr Gly Asp Lys Asp Ser Val Asn Leu Thr Cys Ser Thr Asn Asp
            340                 345                 350
Thr Gly Ile Ser Ile Arg Trp Phe Phe Lys Asn Gln Ser Leu Pro Ser
        355                 360                 365
Ser Glu Arg Met Lys Leu Ser Gln Gly Asn Thr Thr Leu Ser Ile Asn
    370                 375                 380
Pro Val Lys Arg Glu Asp Ala Gly Thr Tyr Trp Cys Glu Val Phe Asn
385                 390                 395                 400
Pro Ile Ser Lys Asn Gln Ser Asp Pro Ile Met Leu Asn Val Asn Tyr
                405                 410                 415
Asn Ala Leu Pro Gln Glu Asn Gly Leu Ser Pro Gly Ala Ile Ala Gly
            420                 425                 430
Ile Val Ile Gly Val Val Ala Leu Val Ala Leu Ile Ala Val Ala Leu
        435                 440                 445
Ala Cys Phe Leu His Phe Gly Lys Thr Gly Arg Ala Ser Asp Gln Arg
    450                 455                 460
Asp Leu Thr Glu His Lys Pro Ser Val Ser Asn His Thr Gln Asp His
465                 470                 475                 480
Ser Asn Asp Pro Pro Asn Lys Met Asn Glu Val Thr Tyr Ser Thr Leu
                485                 490                 495
Asn Phe Glu Ala Gln Gln Pro Thr Gln Pro Thr Ser Ala Ser Pro Ser
            500                 505                 510
Leu Thr Ala Thr Glu Ile Ile Tyr Ser Glu Val Lys Lys Gln
        515                 520                 525

SEQUENCE LISTING

<110> LeukoCom GmbH

<120> A novel antibody binding specifically to human CEACAM1/3/5 and use
thereof

<130> P25629EP

<160> 21

<170> BiSSAP 1.3.6

<210> 1
<211> 8
<212> PRT
<213> Artificial Sequence

<220>
<223> HCDR1

<400> 1
Gly Tyr Thr Phe Thr Thr Tyr Val
1               5

<210> 2
<211> 8
<212> PRT
<213> Artificial Sequence

<220>
<223> HCDR2

<400> 2
Phe Asn Pro Tyr Asn Asp Gly Thr
1               5

<210> 3
<211> 11
<212> PRT
<213> Artificial Sequence

<220>
<223> HCRD3

<400> 3
Ala Arg Trp Ala Tyr Asp Gly Ser Tyr Ala Tyr
1               5                   10

<210> 4
<211> 6
<212> PRT
<213> Artificial Sequence

<220>
<223> LCDR1

<400> 4
Asp His Ile Asn Asn Trp

1               5

<210> 5
<211> 4
<212> PRT
<213> Artificial Sequence

<220>
<223> LCDR2, wherein X at position 4 can be any amino acid

<400> 5
Gly Ala Thr Xaa
1

<210> 6
<211> 9
<212> PRT
<213> Artificial Sequence

<220>
<223> LCDR3

<400> 6
Gln Gln Tyr Trp Arg Thr Pro Phe Thr
1               5

<210> 7
<211> 118
<212> PRT
<213> Artificial Sequence

<220>
<223> VH with HCDR1 to 3

<400> 7
Glu Val Gln Leu Gln Gln Ser Gly Pro Glu Leu Val Lys Pro Gly Thr
1               5                   10                  15
Ser Val Lys Met Ser Cys Lys Ala Ser Gly Tyr Thr Phe Thr Thr Tyr
            20                  25                  30
Val Met His Trp Val Gln Gln Lys Pro Gly Gln Gly Leu Asp Trp Ile
        35                  40                  45
Gly Phe Phe Asn Pro Tyr Asn Asp Gly Thr Lys Tyr Asn Glu Lys Phe
    50                  55                  60
Lys Gly Lys Ala Thr Leu Thr Ser Asp Lys Ser Ser Ser Thr Ala Tyr
65                  70                  75                  80
Met Glu Leu Ser Ser Leu Thr Ser Glu Asp Ser Ala Val Tyr Tyr Cys
                85                  90                  95
Ala Arg Trp Ala Tyr Asp Gly Ser Tyr Ala Tyr Trp Gly Gln Gly Thr
            100                 105                 110
Thr Leu Thr Val Ser Ser
        115

<210> 8
<211> 107
<212> PRT
<213> Artificial Sequence

<220>
<223> VL with LCDR1 to 3

<400> 8
Asp Ile Gln Met Thr Gln Ser Ser Ser Tyr Leu Ser Val Phe Leu Gly
1               5                   10                  15
Gly Arg Val Thr Ile Thr Cys Lys Ala Ser Asp His Ile Asn Asn Trp
            20                  25                  30
Leu Ala Trp Tyr Gln Gln Lys Pro Gly Asn Ala Pro Arg Leu Leu Ile
        35                  40                  45
Ser Gly Ala Thr Ser Leu Glu Thr Gly Val Pro Ser Arg Phe Ser Gly
    50                  55                  60
Ser Gly Ser Gly Lys Asp Tyr Thr Leu Ser Ile Ile Ser Leu Gln Thr
65                  70                  75                  80
Glu Asp Val Ala Thr Tyr Tyr Cys Gln Gln Tyr Trp Arg Thr Pro Phe
                85                  90                  95
Thr Phe Gly Ser Gly Thr Lys Leu Glu Ile Lys
            100                 105

<210> 9
<211> 354
<212> DNA
<213> Artificial Sequence

<220>
<223> encoding VH

<400> 9
gaggtccagc tgcagcaatc tggacctgag ctggtaaagc ctgggacttc agtgaagatg      60

tcctgcaagg cttctggata cacattcact acctatgtta tgcactgggt gcaacagaag     120

cctgggcagg gccttgactg gattggattt tttaatcctt acaatgatgg tactaagtac     180

aatgagaagt tcaaaggcaa ggccacactg acttcagaca aatcctccag cacagcctac     240

atggaactca gcagcctgac ctctgaggac tctgcggtct attactgtgc aagatgggcc     300

tacgatggta gctacgccta ctggggccaa ggcaccactc tcacagtctc ctca           354

<210> 10
<211> 321
<212> DNA
<213> Artificial Sequence

<220>
<223> encoding VL

<400> 10
gatatccaga tgacacaatc ttcatcctac ttgtctgtat ttctaggagg cagagtcacc      60

attacttgca aggcaagtga ccacattaat aattggttag cctggtatca gcagaaacca     120

ggaaatgctc ctaggctctt aatatctggg gcaaccagtt tggaaactgg ggttccttca     180

agattcagtg gcagtggatc tggaaaggat tacactctca gcattatcag tcttcagact     240

gaagatgttg ctacttatta ctgtcaacag tattggagaa ctccattcac gttcggctcg     300

gggacaaagt tggaaataaa a                                               321

<210> 11
<211> 10
<212> PRT
<213> Homo sapiens

<220>
<223> fragment of huCEACAM1, epitope of antibody of the invention

<400> 11
Pro Gln Gln Leu Phe Gly Tyr Ser Trp Tyr
1               5                   10

<210> 12
<211> 526
<212> PRT
<213> Homo sapiens

<220>
<223> CEACAM1

<400> 12
Met Gly His Leu Ser Ala Pro Leu His Arg Val Arg Val Pro Trp Gln
1               5                   10                  15
Gly Leu Leu Leu Thr Ala Ser Leu Leu Thr Phe Trp Asn Pro Pro Thr
            20                  25                  30
Thr Ala Gln Leu Thr Thr Glu Ser Met Pro Phe Asn Val Ala Glu Gly
        35                  40                  45
Lys Glu Val Leu Leu Leu Val His Asn Leu Pro Gln Gln Leu Phe Gly
    50                  55                  60
Tyr Ser Trp Tyr Lys Gly Glu Arg Val Asp Gly Asn Arg Gln Ile Val
65                  70                  75                  80
Gly Tyr Ala Ile Gly Thr Gln Gln Ala Thr Pro Gly Pro Ala Asn Ser
                85                  90                  95
Gly Arg Glu Thr Ile Tyr Pro Asn Ala Ser Leu Leu Ile Gln Asn Val
            100                 105                 110
Thr Gln Asn Asp Thr Gly Phe Tyr Thr Leu Gln Val Ile Lys Ser Asp
        115                 120                 125
Leu Val Asn Glu Glu Ala Thr Gly Gln Phe His Val Tyr Pro Glu Leu
    130                 135                 140
Pro Lys Pro Ser Ile Ser Ser Asn Asn Ser Asn Pro Val Glu Asp Lys
145                 150                 155                 160
Asp Ala Val Ala Phe Thr Cys Glu Pro Glu Thr Gln Asp Thr Thr Tyr
                165                 170                 175
Leu Trp Trp Ile Asn Asn Gln Ser Leu Pro Val Ser Pro Arg Leu Gln
            180                 185                 190
Leu Ser Asn Gly Asn Arg Thr Leu Thr Leu Leu Ser Val Thr Arg Asn
        195                 200                 205
Asp Thr Gly Pro Tyr Glu Cys Glu Ile Gln Asn Pro Val Ser Ala Asn
    210                 215                 220
Arg Ser Asp Pro Val Thr Leu Asn Val Thr Tyr Gly Pro Asp Thr Pro
225                 230                 235                 240
Thr Ile Ser Pro Ser Asp Thr Tyr Tyr Arg Pro Gly Ala Asn Leu Ser
                245                 250                 255
Leu Ser Cys Tyr Ala Ala Ser Asn Pro Pro Ala Gln Tyr Ser Trp Leu
            260                 265                 270
Ile Asn Gly Thr Phe Gln Gln Ser Thr Gln Glu Leu Phe Ile Pro Asn
        275                 280                 285
Ile Thr Val Asn Asn Ser Gly Ser Tyr Thr Cys His Ala Asn Asn Ser
    290                 295                 300
Val Thr Gly Cys Asn Arg Thr Thr Val Lys Thr Ile Ile Val Thr Glu
305                 310                 315                 320

Leu Ser Pro Val Val Ala Lys Pro Gln Ile Lys Ala Ser Lys Thr Thr
                325                 330                 335
Val Thr Gly Asp Lys Asp Ser Val Asn Leu Thr Cys Ser Thr Asn Asp
            340                 345                 350
Thr Gly Ile Ser Ile Arg Trp Phe Phe Lys Asn Gln Ser Leu Pro Ser
        355                 360                 365
Ser Glu Arg Met Lys Leu Ser Gln Gly Asn Thr Thr Leu Ser Ile Asn
    370                 375                 380
Pro Val Lys Arg Glu Asp Ala Gly Thr Tyr Trp Cys Glu Val Phe Asn
385                 390                 395                 400
Pro Ile Ser Lys Asn Gln Ser Asp Pro Ile Met Leu Asn Val Asn Tyr
                405                 410                 415
Asn Ala Leu Pro Gln Glu Asn Gly Leu Ser Pro Gly Ala Ile Ala Gly
            420                 425                 430
Ile Val Ile Gly Val Val Ala Leu Val Ala Leu Ile Ala Val Ala Leu
        435                 440                 445
Ala Cys Phe Leu His Phe Gly Lys Thr Gly Arg Ala Ser Asp Gln Arg
    450                 455                 460
Asp Leu Thr Glu His Lys Pro Ser Val Ser Asn His Thr Gln Asp His
465                 470                 475                 480
Ser Asn Asp Pro Pro Asn Lys Met Asn Glu Val Thr Tyr Ser Thr Leu
                485                 490                 495
Asn Phe Glu Ala Gln Gln Pro Thr Gln Pro Thr Ser Ala Ser Pro Ser
            500                 505                 510
Leu Thr Ala Thr Glu Ile Ile Tyr Ser Glu Val Lys Lys Gln
        515                 520                 525

<210> 13
<211> 12
<212> PRT
<213> Homo sapiens



<220>
<223> S6

<400> 13
Lys Glu Val Leu Leu Leu Val His Asn Leu Pro Gln
1               5                   10

<210> 14
<211> 12
<212> PRT
<213> Homo sapiens



<220>
<223> S7

<400> 14
Leu Leu Leu Val His Asn Leu Pro Gln Gln Leu Phe
1               5                   10

<210> 15
<211> 12
<212> PRT
<213> Homo sapiens



<220>
<223> S8

<400> 15

Val His Asn Leu Pro Gln Gln Leu Phe Gly Tyr Ser
1               5                   10

<210> 16
<211> 12
<212> PRT
<213> Homo sapiens


<220>
<223> S9

<400> 16
Leu Pro Gln Gln Leu Phe Gly Tyr Ser Trp Tyr Lys
1               5                   10

<210> 17
<211> 12
<212> PRT
<213> Homo sapiens


<220>
<223> S10

<400> 17
Gln Leu Phe Gly Tyr Ser Trp Tyr Lys Gly Glu Arg
1               5                   10

<210> 18
<211> 12
<212> PRT
<213> Homo sapiens


<220>
<223> S11

<400> 18
Gly Tyr Ser Trp Tyr Lys Gly Glu Arg Val Asp Gly
1               5                   10

<210> 19
<211> 12
<212> PRT
<213> Homo sapiens


<220>
<223> S12

<400> 19
Trp Tyr Lys Gly Glu Arg Val Asp Gly Asn Arg Gln
1               5                   10

<210> 20
<211> 12
<212> PRT
<213> Homo sapiens


<220>
<223> S13

<400> 20
Gly Glu Arg Val Asp Gly Asn Arg Gln Ile Val Gly
1               5                   10

<210> 21
<211> 12
<212> PRT
<213> Homo sapiens

<220>
<223> S14

<400> 21
Val Asp Gly Asn Arg Gln Ile Val Gly Tyr Ala Ile
1               5                   10